# EUROPEAN PATENT APPLICATION

(11) **EP 4 195 218 A1**
(43) Date of publication of application: **14.06.2023**
(21) Application number: 22209830.3
(22) Date of filing: 28.11.2022
(51) Int. Cl.: G16H 40/20, G16H 40/63, G16H 50/20, G16H 50/30, A61B 5/00

(54) **METHODS AND SYSTEMS FOR PATIENT MANAGEMENT**

(30) Priority: 08.12.2021 US 202117643381
(71) Applicant: GE Precision Healthcare LLC, Wauwatosa, WI 53226 (US)
(72) Inventor: MANCL, Ryan, Grapevine, 76051 (US); TERRY, Jeffrey Richardson, Dallas, 75205 (US); FIXLER, Tamas, Thornhill, L4J 4Y2 (CA); MARTIN, Kathleen, Pinckney, 48169 (US); PANDA, Pravat, Hanover Park, 60133 (US); CHANG, Jehoshua Joon, Suwanee, 30024 (US)
(74) Representative: Kilburn & Strode LLP

(57) **Abstract**

Various methods and systems are provided for automatically generating a recommendation to downgrade a patient from an intensive care unit (ICU) to a different medical unit. In one embodiment, a method for a patient downgrade recommendation system comprises, for each patient of a plurality of selected patients of the ICU, selecting (454) a set of downgrade criteria for the patient; from a first, larger set of patient data of the patient, automatically compiling (458) a second, smaller set of patient data based on the set of downgrade criteria; applying (468) a set of rules associated with the set of downgrade criteria to the second, smaller set of patient data to generate a downgrade recommendation for the patient; and arranging (408) the graphical downgrade recommendation elements of the plurality of selected patients in a single integrated view of a user interface on a display device of the patient downgrade recommendation system.

## Description

### FIELD

Embodiments of the subject matter disclosed herein relate to managing patients of an intensive care unit, and more particularly, to monitoring patient status.

### BACKGROUND

A patient suffering from acute symptoms or conditions may be treated in an intensive care unit (ICU), where the patient may be provided with a greater amount of care, medical resources, and treatment options than other medical units. When an ICU patient's condition improves, a status of the ICU patient may be downgraded and the patient may be transferred from the ICU to a different medical unit for continued monitoring and/or treatment. Because a higher cost is associated with the greater amount of care, medical resources, and treatment options of the ICU, transferring the patient to the different medical unit may reduce an overall cost of treating the patient. Additionally, a demand for resources of the ICU (e.g., beds, ventilators, etc.) may be high, whereby providing appropriate care to a continuous stream of patients with serious medical conditions may include regularly downgrading patients to free up the resources.

An important responsibility of care providers working in an ICU is determining which patients may be downgraded, and when the patients are stable enough to be transferred. However, reviewing patient records in an EHR system to determine whether a patient may be downgraded may be laborious and time consuming. The patient data to be reviewed may include numerous medical procedures and records generated during investigations of the patient, including a variety of examinations such as blood tests, urine tests, pathology reports, image-based scans, etc. The data may be stored in an Electronic Health Record (EHR) system, which may comprise, or aggregate information from a plurality of different computer systems and databases. Sorting and extracting information may be a slow and inefficient process, increasing a likelihood of missing records with relevant data which may be spread out across a large number of less informative records. Further, a cognitive load on a care provider may be high, because the care provider may have to compare statuses of a plurality of patients. Determining the status of each patient may include remembering patient data accessed from a variety of sources.

As a result, determining and monitoring patient statuses in an ICU may consume a large proportion of a healthcare professional's time, consequently reducing an amount of time available for tending to patients. Additionally, inefficiencies and risks of incorrectly estimating patient statuses when downgrading the patient may increase a likelihood of poor patient outcomes. These issues may become increasingly problematic when a demand for ICU resources increases, such as in cases of pandemics or mass casualty events.

### BRIEF DESCRIPTION

In one embodiment, a method for a patient downgrade recommendation system of an intensive care unit (ICU) includes, for each patient of a plurality of selected patients of the ICU, selecting a set of downgrade criteria for the patient; from a first, larger set of patient data of the patient accessible from a plurality of sources communicably coupled to the computer system, automatically compiling a second, smaller set of patient data based on the set of downgrade criteria; applying a set of rules associated with the set of downgrade criteria to the second, smaller set of patient data to generate a downgrade recommendation on whether the patient may be downgraded from a first status to a second, lower status, the first status and second status based on one or more medical conditions of the patient; generating a graphical downgrade recommendation element summarizing the downgrade recommendation; arranging the graphical downgrade recommendation elements of the plurality of selected patients in a single integrated view of a user interface (UI) of the patient downgrade recommendation system; and displaying the UI on a display device of the patient downgrade recommendation system.

It should be understood that the brief description above is provided to introduce in simplified form a selection of concepts that are further described in the detailed description. It is not meant to identify key or essential features of the claimed subject matter, the scope of which is defined uniquely by the claims that follow the detailed description. Furthermore, the claimed subject matter is not limited to implementations that solve any disadvantages noted above or in any part of this disclosure.

### BRIEF DESCRIPTION OF THE DRAWINGS

The present invention will be better understood from reading the following description of non-limiting embodiments, with reference to the attached drawings, wherein below:
FIG. 1 illustrates an existing system for displaying clinical information of a patient to a user;
FIG. 2 illustrates a patient downgrade recommendation system, which may automatically generate patient downgrade recommendations for a care provider in accordance with an aspect of the disclosure;
FIG. 3 shows an example user interface of the downgrade recommendation system of FIG. 2 in accordance with an aspect of the disclosure;
FIGS. 4A and 4B are flowcharts for generating downgrade recommendations according to an aspect of the disclosure;
FIG. 5A and FIG. 5B show example pop-up display panels of the user interface of the downgrade recommendation system of FIG. 2;
FIG. 6 shows an exemplary set of readiness indicators that may be displayed for a patient awaiting a transfer;
FIG. 7 shows another example pop-up display panel of the user interface of the downgrade recommendation system of FIG. 2; and
FIG. 8 shows a method for displaying a downgrade recommendation graphical user interface.

### DETAILED DESCRIPTION

The following description relates to systems and methods for managing patients in care units of a hospital or healthcare organization. Efficient patient management may include stabilizing patients with serious medical conditions and providing initial treatment in a first care unit, such as an intensive care unit (ICU), and once the patient is stable, downgrading the patient to an appropriate second care unit for monitoring, where a level and a cost of care of the second care unit may be lower. By maintaining a regular flow of patients from the first care unit into appropriate second care units, resources of the first care unit (e.g., beds, ventilators, etc.) may be made continuously available for new patients with serious conditions.

Efficient patient management of an ICU may depend on an efficient process for regularly assessing a status of a plurality of patients of the ICU. However, determining a latest status of a patient may be challenging due to a large amount of patient data to review. The amount of generated health data rose 10x in the last seven years, and is on the rise due to technological advancements. As one example, the medical history of a 5-year breast cancer relapse patient with scattered data will comprise of at least 1500-3000 records. Figuring out what a journey was for the patient (diagnosis, staging, treatment, treatment response, etc.), how the patient responded to treatment, and how the journey affects a current state may be challenging, as it may rely on a care provider sifting through a large volume of health data collected for the patient.

Patient data may be stored in one or more electronic health record (EHR) systems. Different types of patient data may be stored in different locations, accessed via different interfaces, and used in different ways to make decisions about patient care. The medical data may include diagnostic data, pathology data, consultation data, and treatment data, which may be accessed in different ways. In some examples, a care provider may consult diagnostic data by accessing a first system, pathology data by accessing a second system, consultation data by accessing a third system, and treatment data by accessing a fourth system. In other examples, the diagnostic data, pathology data, consult data, and treatment data may be accessible via a single EHR system, but a user may have to perform a different set of actions (e.g., entering in search terms, selecting controls, etc.) from different reference points to view each different type of data. Viewing and considering such data independently when making patient care decisions may lead to overlooked information that may affect a treatment decision, and may increase time burden and mental load placed on the clinicians caring for a patient.

As disclosed herein, the issues described above may be addressed by an AI-based, computerized patient downgrade recommendation system that automatically retrieves and aggregates data relevant to health conditions of a plurality of patients, and generates recommendations regarding which patients may be eligible for a status downgrade. As used herein, a downgrade recommendation refers to a recommendation, generated for an individual patient, for a recommended action to take with respect to potential downgrade of the patient from a first status to a second status, where patients of the first status are treated in a first type of care unit (e.g., an ICU) for patients with more serious conditions, and patients of the second status are treated in a second type of care unit (e.g., a floor unit) for patients with less serious conditions. The downgrade recommendation may be that the patient is ready to be downgraded, that the patient is not ready to be downgraded, that the patient may possibly be downgraded, or a different recommendation. The automatic generation and display of patient downgrade recommendations may reduce an amount of time spent by a care provider in determining statuses of a rotating body of patients, thereby increasing an amount of time available to the care provider to attend to patients, and allow a greater number of criteria to be considered in making a downgrade recommendation. Further, a cognitive load placed on the care provider may also be reduced, thereby reducing a risk of error in assessing patient status.

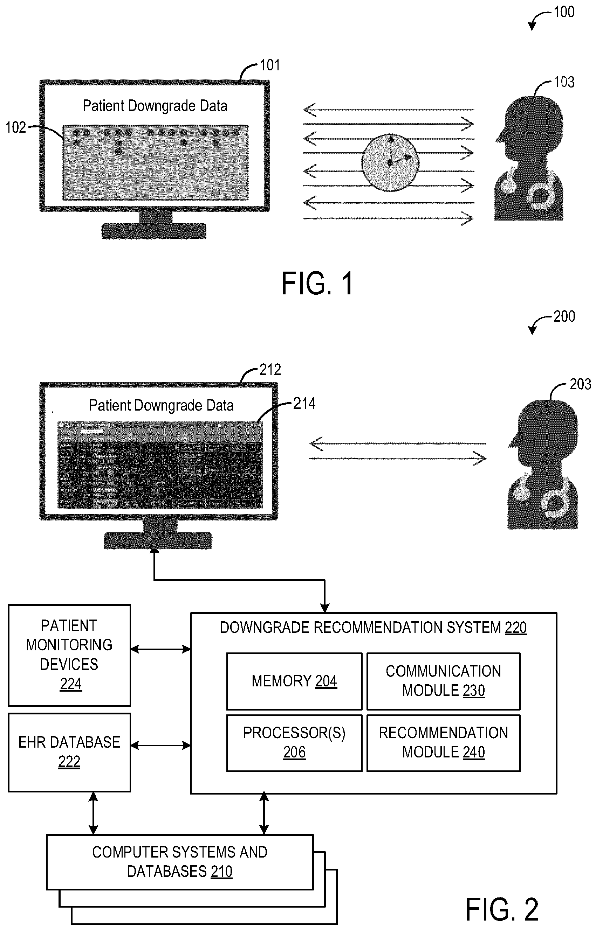

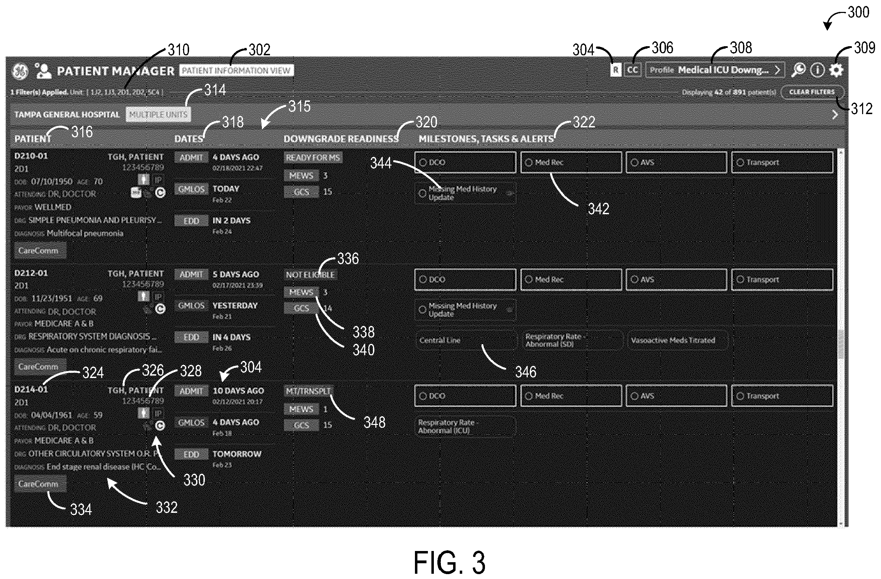

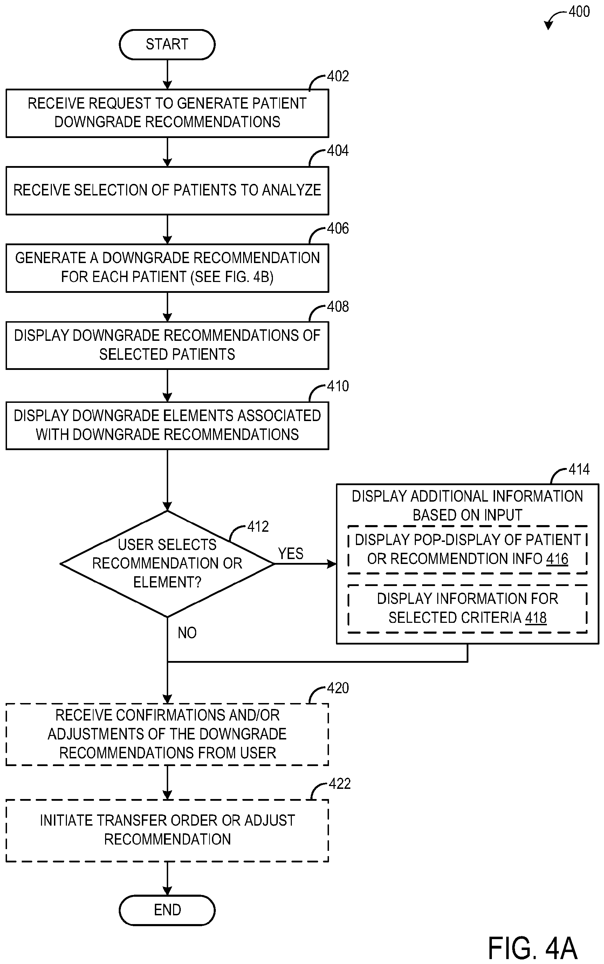

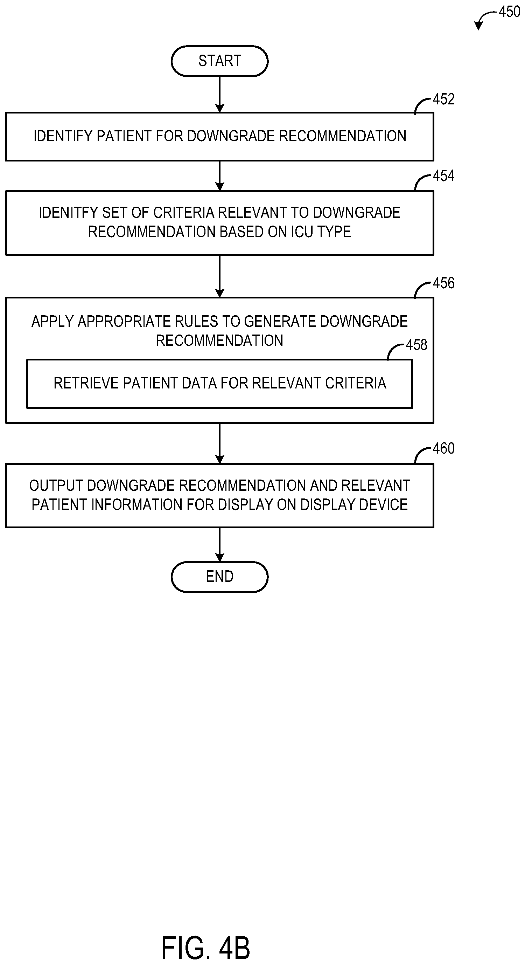

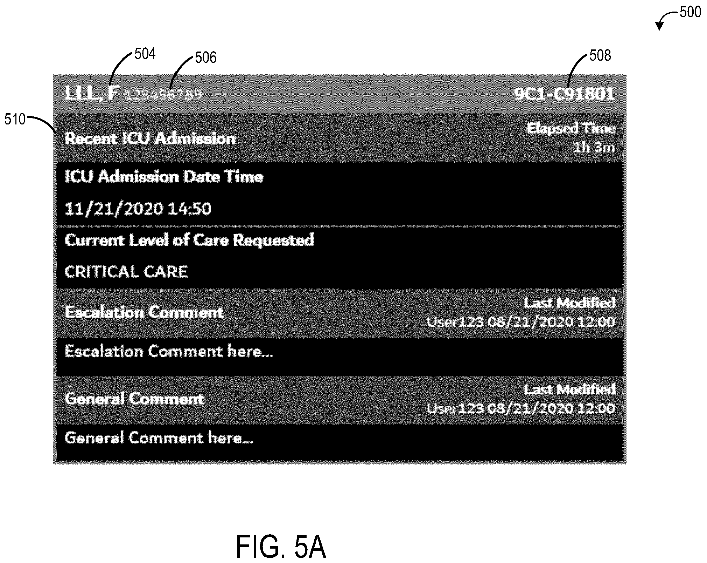

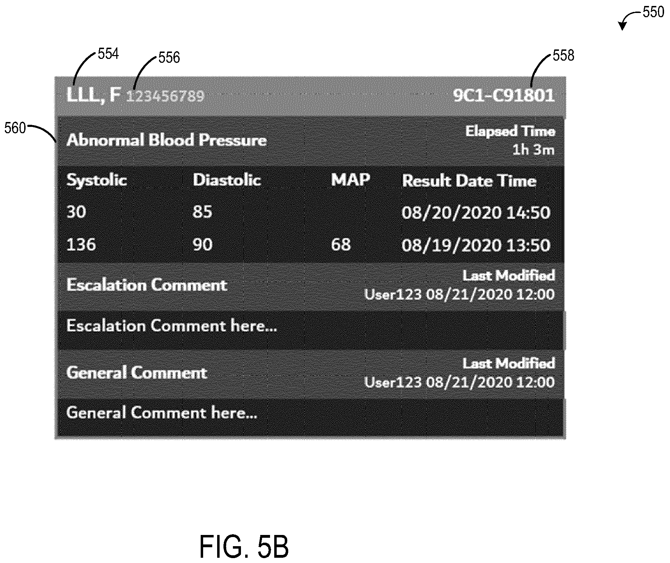

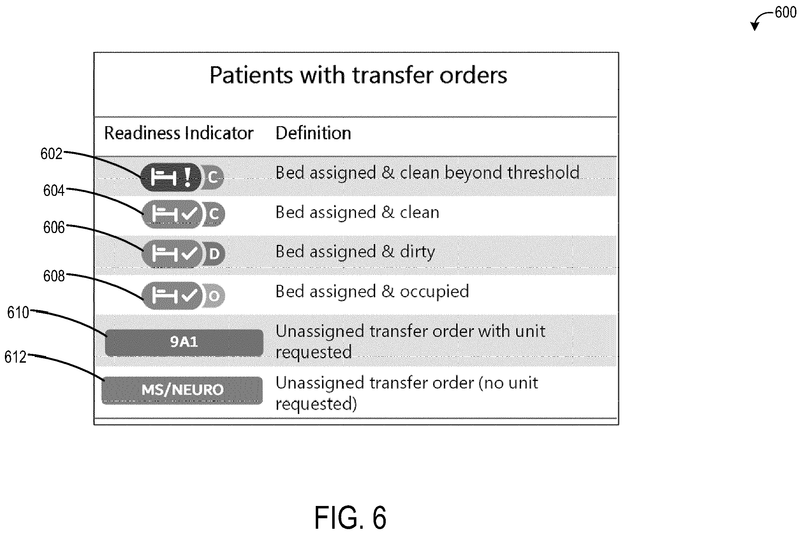

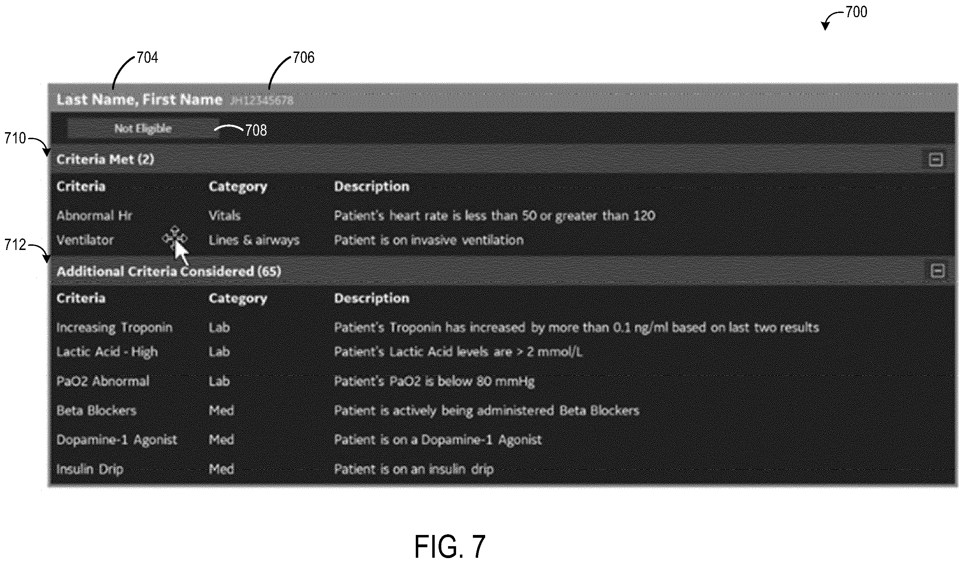

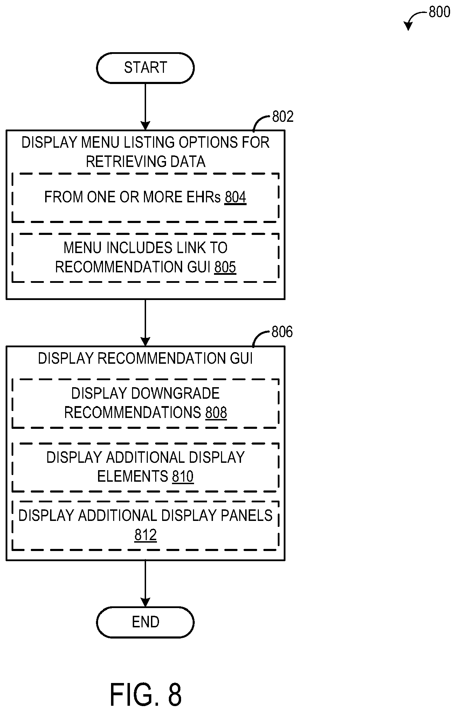

Embodiments of the present disclosure will now be described, by way of example, with reference to the figures. FIG. 1 shows an existing system for displaying clinical information relevant to determining a status of a patient to a user in a first, time-consuming manner. FIG. 2 shows a proposed patient downgrade recommendation system, as described herein, for displaying patient downgrade recommendations and associated clinical information to a user in a second, more efficient manner where a care provider may not have to consult individual patient records from a variety of sources. FIG. 3 shows an exemplary user interface (UI) of the patient downgrade recommendation system, which may include one or more additional display panels such as the pop-up display panels of FIG. 5A, FIG. 5B, and FIG. 7. Various icons may be displayed in the UI, such as the icons shown in FIG. 6. The patient downgrade recommendations that are displayed in the UI may be generated by following one or more steps of procedures such as the methods of FIG. 4A and FIG. 4B and may be displayed according to the method of FIG. 8.

Referring now to FIG. 1, an existing system 100 is shown for displaying clinical information 102 of a patient to a user 103, where the clinical information may support a decision regarding whether to downgrade a status of the patient. As described above, downgrading a status of the patient may be a prerequisite for transferring the patient from an ICU of a medical care system to a different care unit (e.g., a relevant floor unit of a hospital) in order to create space for a new patient with a more serious medical condition.

System 100 may include a display 101 on which the clinical information 102 may be displayed. The display 101 may be any known device having a screen to display the clinical information 102. The clinical information 102 as shown in FIG. 1 may be stored in memory of a computer system (not shown), and or may be retrieved from one or more systems or databases communicatively coupled to the computer system. The clinical information 102 may be generated during investigations of the patient (e.g., clinical visits, examinations, tests) and the clinical information 102 may include a variety of medical records including examination reports, blood tests, urine tests, pathology reports, and scans using various medical imaging systems such as ultrasound, magnetic resonance imaging, computed tomography systems, and other radiological investigations. These medical records generated from various investigations may be stored in different formats. For example, the medical images may be stored in a digital imaging in medical communication (DICOM) format that is different than the format for storing the blood parameter reports which is still different than the format of storing the pathological reports.

Accordingly, when the user 103 evaluates a current status of the patient and patient history in order to determine whether the status of the patient may be downgraded to a different status (e.g., indicating a less serious medical condition), the user 103 may undergo a time-consuming and mentally burdensome process where the user 103 manually selects medical records of the clinical information 102 to review. As mentioned previously, the number of relevant medical records may be large, and may be interspersed with medical records from routine or other visits that may or may not be relevant to the current status of the patient. For the user 103, figuring out the patient's status, how the patient is responding to treatment, and a relationship of the patient's medical history to the current patient status may be time-consuming and may place a large cognitive load on the user. Accordingly, the amount of time a user must devote to reviewing a patient's medical history may be large, thus reducing the number of patients the user may attend to and/or reducing an amount of time available to the user to carry out other patient treatment or management tasks. Further, without any mechanism to help the user quickly hone in on relevant information, information may be missed that may result in an inappropriate downgrade, compromising patient care, or may result in a patient remaining in an ICU when the patient could be adequately treated in a different unit, reducing a cost to the patient and freeing up ICU resources for other patients in need of intensive care. Further still, the existing system forces the user to navigate through individual medical records, which may result in the user selecting and reviewing medical records that are not relevant, selecting unwanted medical records in error, etc., which may make the computing system itself inefficient because the computing system may be asked to retrieve and display more information than necessary.

Thus, according to embodiments disclosed herein, a downgrade recommendation system may be employed to retrieve and compile relevant patient status information from a variety of sources, and automatically generate and display downgrade recommendations for selected patients (e.g., patients of an ICU). The downgrade recommendations may summarize and present a limited amount of patient data relevant to a status downgrade, thereby facilitating an efficient and timely decision regarding which patients may be downgraded to a lower-cost care unit. The generation and display of the downgrade recommendations may reduce a footprint of the patient medical data and may reduce the cognitive load on the user/clinician, while highlighting the major health issues affecting patients.

FIG. 2 shows a medical information system 200 according to an aspect of the disclosure. The medical information system 200 may include a downgrade recommendation system 220 configured for generating patient downgrade recommendations and associated clinical information of a patient that may be displayed to a user 203 via a display 212. The display 212 may be any known device having a screen to display the patient downgrade recommendations and associated clinical information. The display device 212, and in some examples, more than one display device, may be communicatively coupled to downgrade recommendation system 220. The display device 212 may include a processor, memory, communication module, user input device, display (e.g., screen or monitor), and/or other subsystems and may be in the form of a desktop computing device, a laptop computing device, a tablet, a smart phone, or other device. The display device 212 may be adapted to send and receive encrypted data and display medical information, including medical images in a suitable format such as digital imaging and communications in medicine (DICOM) or other standards. The display device 212 may be located locally at a medical facility (such as in an ICU of the medical facility) and/or remotely from the medical facility (such as a care provider's mobile device).

The patient downgrade recommendations may be viewed via a user interface (UI) 214 (which may be a graphical user interface and thus also referred to as a GUI) of the display device 212. When viewing the patient downgrade recommendations via the UI 214, a care provider may enter input (e.g., via a user input device, which may include a keyboard, mouse, microphone, touch screen, stylus, or other device) that may be processed by the downgrade recommendation system 220. In examples where the user input is a selection of a link or control button of the UI 214, the user input may trigger display of additional clinical information relevant to one or more patient downgrades, or other actions.

The medical information system 200 may include an EHR database 222, which may be communicatively coupled to the downgrade recommendation system 220. EHR database 222 may be stored in a mass storage device configured to communicate with secure channels (e.g., HTTPS and TLS), and store data in encrypted form. Further, the EHR database/computing system may be configured to control access to patient electronic medical records such that only authorized healthcare providers may edit and access the electronic health records. An EHR for a patient may include medical device data (e.g., vital signs measured or otherwise ascertained by medical devices such as heart rate, oxygen saturation, etc.), user-specified medical parameters (e.g., acuity scores, pain scores), patient demographic information, family medical history, past medical history, lifestyle information, preexisting medical conditions, current medications, allergies, surgical history, past medical screenings and procedures, past hospitalizations and visits, etc.

In addition to the EHR database 222, the patient information used to generate the downgrade recommendations may also be stored in one or more computer systems and databases 210 in communication with downgrade recommendation system 220 and/or the EHR database 222. For example, the computer systems and databases 210 may include a picture archiving and communication system (PACS) that may store and communicate medical images and associated reports (e.g., clinician findings), such as ultrasound images, MRI images, etc., according to the DICOM format. The computer systems and databases 210 may include a radiology information system (RIS), which may store radiology images and associated reports, such as CT images, X-ray images, etc. The computer systems and databases 210 may include a pathology database, which may store pathology images and related reports, including visible light or fluorescence images of tissue, such as immunohistochemistry (IHC) images. It should be appreciated that the example computer systems and databases provided herein are for illustrative purposes, and in various embodiments, additional, fewer, or different computer systems and/or databases may be included without departing from the scope of this disclosure.

Downgrade recommendation system 220 may include a memory 204 and a processor(s) 206 to store and execute the methods disclosed herein to generate the downgrade recommendations and patient status data, as well as send and receive communications, graphical user interfaces, medical data, and other information. Memory 204 may include one or more data storage structures, such as optical memory devices, magnetic memory devices, or solid-state memory devices, for storing programs and routines executed by processor(s) 206 to carry out various functionalities disclosed herein. Memory 204 may include any desired type of volatile and/or non-volatile memory such as, for example, static random access memory (SRAM), dynamic random access memory (DRAM), flash memory, read-only memory (ROM), etc. Processor(s) 206 may be any suitable processor, processing unit, or microprocessor, for example. Processor(s) 206 may be a multi-processor system, and, thus, may include one or more additional processors that are identical or similar to each other and that are communicatively coupled via an interconnection bus.

In some examples, downgrade recommendation system 220 may be implemented over a cloud or other computer network. For example, downgrade recommendation system 220 is shown in FIG. 2 as constituting a single entity, but it should be appreciated that downgrade recommendation system 220 may be distributed across multiple devices, such as across multiple servers.

Downgrade recommendation system 220 may be configured to generate and output one or more patient downgrade recommendations for display via display 212. Each patient downgrade recommendation may include additional clinical information and/or clinical markers (also referred to as criteria) relevant to the downgrade recommendation, as described in greater detail below in reference to FIGS. 3-8. The clinical information may be automatically extracted from the EHRs and/or other medical information of the patient (e.g., pathology reports, imaging scans/reports, etc.) that may or may not be stored as EHRs. The extracted clinical information may be assembled into a natural-language-like format and displayed along with and/or adjacent to a relevant downgrade recommendation, as described in greater detail below in reference to FIG. 3. Generating patient downgrade recommendations may reduce a time spent by the user reading information for a current patient that is not relevant to a downgrade, allowing the user to view information for a plurality of patients with increased efficiency.

As used herein, the term "module" may include a hardware and/or software system that operates to perform one or more functions. For example, a module may include a computer processor, controller, or other logic-based device that performs operations based on instructions stored on a tangible and non-transitory computer readable storage medium, such as a computer memory. Alternatively, a module may include a hard-wired device that performs operations based on hard-wired logic of the device. Various modules shown in the attached figures may represent the hardware that operates based on software or hardwired instructions, the software that directs hardware to perform the operations, or a combination thereof.

"Modules" may include or represent hardware and associated instructions (e.g., software stored on a tangible and non-transitory computer readable storage medium, such as a computer hard drive, ROM, RAM, or the like) that perform one or more operations described herein. The hardware may include electronic circuits that include and/or are connected to one or more logic-based devices, such as microprocessors, processors, controllers, or the like. These devices may be off-the-shelf devices that are appropriately programmed or instructed to perform operations described herein from the instructions described above. Additionally or alternatively, one or more of these devices may be hard-wired with logic circuits to perform these operations.

Downgrade recommendation system 220 may include a communication module 230. Communication module 230 may facilitate transmission of electronic data within and/or among one or more systems. Communication via communication module 230 can be implemented using one or more protocols. In some examples, communication via communication module 230 occurs according to one or more standards (e.g., Digital Imaging and Communications in Medicine (DICOM), Health Level Seven (HL7), ANSI X12N, etc.). Communication module 230 can be a wired interface (e.g., a data bus, a Universal Serial Bus (USB) connection, etc.) and/or a wireless interface (e.g., radio frequency, infrared, near field communication (NFC), etc.). For example, communication module 230 may communicate via wired local area network (LAN), wireless LAN, wide area network (WAN), etc. using any past, present, or future communication protocol (e.g., BLUETOOTH^{™}, USB 2.0, USB 3.0, etc.).

Downgrade recommendation system 220 may include a recommendation module 240. The recommendation module 240 may store one or more models that may be used to generate the downgrade recommendations. The one or more models may include a rules-based system, where machine-implementable disqualification rules constructed around downgrade criteria may be applied to a set of patient data relating to the downgrade criteria retrieved by the downgrade recommendation system 220. For example, one criterion may be blood pressure, and a rule corresponding to the criterion of blood pressure may be that if a blood pressure measurement of a patient is outside a pre-established range of values, then the patient may not be eligible for a downgrade.

Thus, to determine whether a patient may be eligible for a downgrade, a set of downgrade criteria may be selected (e.g., based on factors such as a type of ICU the patient is in), patient data corresponding to the set of downgrade criteria may be retrieved, and a set of rules corresponding to the set of downgrade criteria may be applied to the retrieved patient data. If all the rules are applied, and the conditions imposed by each rule are met, then the recommendation module 240 may generate a recommendation to downgrade the patient. Alternatively, if one or more conditions imposed by a rule are not met by the patient data, the recommendation module 240 may generate a recommendation not to downgrade the patient. In this way, the recommendation module 240 may perform the task of retrieving patient data relating to the selected set of downgrade criteria from the EHR database 222 and the computer systems and databases 210, and analyzing the retrieved data for the care provider, thereby advantageously reducing an amount of time spent by the care provider finding and analyzing data. Further, by structuring the rules as exclusionary rules such that a recommendation to downgrade is generated only if no criteria exists to block the downgrade recommendation, an efficient processing of applying the retrieved data to the criterion is achieved. For example, once a rule is triggered to block the downgrade recommendation for a particular patient, no further data collection and/or rule analysis is immediately required for that particular patent with the current data, but can be performed in background processing to improve computational efficiency.

While not specifically shown in FIG. 2, additional devices described herein (e.g., display device 212) may likewise include user input devices, memory, processors, and communication modules/interfaces similar to communication module 230, memory 204, and processor(s) 206 described above, and thus the description of communication module 230, memory 204, and processor(s) 206 likewise applies to the other devices described herein. As an example, the display device 212 may store user interface templates in memory that include placeholders for relevant information stored on downgrade recommendation system 220 or received via downgrade recommendation system 220, which a user of display device 212 may configure. The downgrade recommendations and relevant patient information may be retrieved from downgrade recommendation system 220 and inserted in the placeholders. The user input devices may include keyboards, mice, touch screens, microphones, or other suitable devices.

Turning to FIG. 3, a first embodiment of a graphical UI 300 is shown of a patient downgrade recommendation system such as the UI 214 of the patient downgrade recommendation system 220 of FIG. 2. UI 300 may be displayed on a display device (e.g., display device 212) of the patient downgrade recommendation system during operation in an ICU. Specifically, UI 300 may be displayed to a care provider of the ICU when the care provider uses the patient downgrade recommendation system to generate downgrade recommendations for patients of the ICU, as described in greater detail below in reference to FIGS. 4A and 4B. The downgrade recommendations may be used in order to transfer one or more patients out of the ICU, when indicated. UI 300 may include a plurality of different individual downgrade recommendations for a respective plurality of patients, which may be displayed in rows of UI 300.

The UI 300 may be displayed in different alternative views. In some embodiments, the UI 300 may be displayed either in an enhanced or rounding view, where information is displayed in a fixed format that is not customizable by the user, or in a condensed or command center view, where less information is displayed in a variable format that is customizable by the user. The user may customize the condensed view by hiding one or more columns, rows, sections, or elements of the enhanced view. For example, a first user may view the UI 300 in a first condensed view that is customized to hide a first portion of patient data displayed in the UI 300, in order to view more rows (e.g., patients) in a single screen. A second user may view the UI 300 in a second condensed view that is customized to hide a column of the UI 300, in order to better view data displayed in a different column. A third user may view the UI 300 in a third, enhanced view, in order to view a comprehensive set of data. Each of the first user, the second user, and the third user may select a desired view and/or switch between desired views by selecting one or more controls of the UI 300, such as a rounding view toggle switch 304 or a command center view toggle switch 306.

The UI 300 may include a hospital unit selection element 314, which may set a scope of the patient downgrade recommendation system. In some embodiments, the hospital unit selection element 314 may comprise a drop-down list of hospital units, where each suitable hospital unit may be included as an item of the drop-down list that may be selected by a user. Suitable hospital units may include various types of ICUs, including pediatric ICUs, neo-natal ICUs, and/or ICUs for different types of medical conditions (neuroscience ICU, cancer ICU, etc.). Suitable hospital units may also include other types of specialty hospital units where a greater or more specific level of care is provided than may be provided at a typical floor care unit of a hospital. Using hospital unit selection element 314, the user may select a desired hospital unit or choose to see an aggregate view of all hospital units in a hospital, hospital network, or healthcare organization.

In some embodiments, when a hospital unit (e.g., an ICU) is selected, a display panel 315 may be automatically updated to generate and show downgrade recommendations for patients of the selected hospital unit. In other embodiments, the display panel 315 may not be automatically updated, and the user may initiate display of downgrade recommendations by selecting a different control element (not shown in FIG. 3), such as a "display downgrade recommendations" button.

When a hospital or suitable hospital unit is selected via the hospital unit selection element 314, a name or identifier of the hospital or suitable hospital unit may be displayed in a profile element 308 of the UI 300. Via the profile element 308, the user may view additional information about the selected hospital or suitable hospital unit. Additionally, in some embodiments, one or more filters may be applied to the UI 300 to filter patient data shown in the UI 300. The one or more filters may be selected, for example, via controls accessible via a settings button 309. A summary of one or more filters applied may be displayed in the filter bar 310, and a user may clear the one or more filters via a clear filters button 312 included in the filter bar 310.

As one example, in response to a pandemic where numerous patients are afflicted with a particular condition (e.g., COVID), temporary recovery units may be established outside of a hospital system or with resources shared between hospital systems. A physician may wish to view downgrade recommendations for patients across all hospital units who suffer from the particular condition, in order to determine how many patients may be eligible to transfer to the temporary recovery units. Using the one or more filters, the user may enter in the particular condition, and select a "display downgrade recommendations" button to view the downgrade recommendations.

As another example, a new geriatric care unit may open at a hospital, and a care provider at an ICU may wish to determine which patients of the ICU may be eligible for transfer to the new geriatric care unit. The care provider may use the one or more filters to specify a threshold age, over which ICU patients would be eligible for treatment at the new geriatric care unit.

In the embodiment shown in FIG. 3, the downgrade recommendations may be displayed in rows of UI 300, where each row corresponds to a patient of the selected hospital unit. The downgrade recommendations and other information may be displayed in columns of UI 300. In other embodiments, a layout of the information may be different. For example, in other embodiments, each patient of the selected hospital unit may be displayed in a separate column, and the downgrade recommendations and other information may be displayed in rows of UI 300. It should be appreciated that the layout and juxtaposition of the elements of UI 300 may vary in different embodiments, and the elements may appear in different visual configurations without departing from the scope of this disclosure. Additionally, not all of the elements shown in UI 300 may be included in an embodiment, and some embodiments may include a greater or lesser number of elements.

UI 300 may include a patient info column 316, which may show identifying and general information of a patient. For each row of UI 300, a patient data panel 332 that includes patient information may be displayed for a corresponding patient in the patient info column 316. The patient data panel 332 may include a patient name (or abbreviated name) 326 and/or an identification number 328. A location code 324 may be included in the patient data panel 332, which may indicate a current location of the patient in the hospital or hospital system (e.g., the ICU to which the patient has been assigned and/or is currently registered). The patient data panel 332 may also include other general information, such as, for example, an age and/or date of birth of the patient, an attending physician of the patient, patient insurance information, a diagnosis of the patient, and the like. Further, in the example shown, some aspects of patient information (such as whether a patient is a new admission or a readmission) may be visualized via icons, such as icon 330.

In some embodiments, a care communication button 334 may be included in the patient data panel 332, which when selected may generate an alert that may be sent to other care providers and/or saved as part of the patient's EHR to indicate that an ordered or commanded patient task has yet to be initiated or completed. However, in some examples, the care communication button 334 may be omitted or may trigger other types of communication or alerts.

UI 300 may include a dates column 318, which may show a timeline of events relating to changes in a status of the patient during a time spent by the patient in the ICU, including dates and times of the events. In some embodiments, the timeline may begin at a time of admission of a patient, and may include dates and times of expected discharge, such as a projected discharge date (e.g., an expected discharge data, EDD) and/or a mean length of stay for patients with the same condition as the patient (e.g., a geometric mean length of stay, GMLOS).

UI 300 may include a downgrade readiness column 320, which may include a downgrade recommendation 336 for a patient (e.g., visually indicating a recommendation on whether the patient may be downgraded in preparation for a transfer to a different care unit or not). In some embodiments, a set of possible downgrade recommendations may be pre-defined, where downgrade recommendation 336 may be a most appropriate downgrade recommendation element based on the set of possible downgrade recommendations. The patient downgrade recommendation system may assign the most appropriate downgrade recommendation based on applying a set of rules corresponding to different patient data according to various downgrade criteria, as described in greater detail below in reference to FIGS. 4A and 4B.

For example, in some embodiments, the set of possible downgrade recommendations 336 may include a "Ready for MS" recommendation which may indicate that a patient is eligible for a transfer to another unit, herein the medical-surgical floor/unit; a "Ready for SD" recommendation, which may indicate that the patient is eligible to be transferred to an intermediate "step-down" unit; a "Possible Downgrade" recommendation, which may indicate that the patient may be eligible for a transfer, pending one or more criteria being met that may be predicted to be met soon; a "Lateral Transfer" recommendation, which may indicate that a patient may be eligible for a lateral transfer to a different ICU or unit with a similar level of care; and a "Not Eligible" recommendation, which may indicate that the patient is not eligible for a downgrade. In other embodiments, fewer, additional, or other recommendations may be used.

In some embodiments, the patients displayed in the rows of UI 300 may be ranked by downgrade recommendation, as shown in FIG. 3. For example, patients receiving a downgrade recommendation of "Ready for MS" may be displayed towards or at the top of a list of selected patients, followed by patients receiving a downgrade recommendation of "Ready for SD", and so on, with patients receiving a downgrade recommendation of "Not Eligible" being displayed towards or at the bottom of the list of selected patients. Additional patients not shown in the current UI view may be viewed by scrolling or another suitable input.

The downgrade recommendations 336 may be presented as color-coded visual elements in order to be more rapidly distinguishable. For example, the "Ready for MS" downgrade recommendation may appear as a green visual element, the "Not Eligible" downgrade recommendation may appear as a red visual element, and other downgrade recommendations 336 may appear in various shades between green and red. Thus, a care provider may be able to quickly scan the UI 300 to determine which patients of a plurality of patients may be eligible for a transfer.

Further, the visual elements may be controls (e.g., buttons) that may be selected by a user via a user input device (e.g., a mouse, a touchscreen, etc.). When the user selects the downgrade recommendation 336, additional information relevant to the downgrade recommendation may be displayed, for example, in a pop-up display panel or on a different screen of the UI 300. Further, in some examples, different information may be displayed when the user enters different user input. In such examples, a first pop-up display panel may be displayed when the user selects the downgrade recommendation with a first type of user input, such as a hover (e.g., where the user positions a cursor or other user input locator over the downgrade recommendation button but does not enter further user input). A second pop-up display panel may be displayed when the user selects the downgrade recommendation with a second type of user input, such as selecting the downgrade recommendation button with one or more clicks or touches.

For example, FIG. 5A shows an example pop-up display panel 500 of the UI 300, which may be displayed in response to the user selecting a downgrade recommendation on the display screen with a first type of user input. Pop-up display panel 500 may include additional information relating to the patient, who may be referenced by a name 504 and/or an identification number 506 (e.g., patient name 326 and/or identification number 328) of the pop-up display panel 500. A location code 508 (e.g., location code 324) indicating where the patient is being treated may also be included in the pop-up display panel 500. The additional information may be displayed in one or more informational rows 510, and may include for example, a date, time, and/or duration of admission, a current level of care, any escalation and/or general comments entered in by a care provider, as well as any other relevant/desired information. In some embodiments, pop-up display panel 500 may include additional controls, such as buttons that when selected, may display different or additional information, and/or may generate additional display panels.

As another example, FIG. 5B shows an example pop-up display panel 550 of the UI 300, which may be displayed in response to the user selecting a downgrade element, such as a downgrade alert 346, on the display screen with a first type of user input. Pop-up display panel 550 may include additional information relating to the patient and downgrade criterion specified by the downgrade element. The patient may be referenced by a name 554 and/or an identification number 556 (e.g., patient name 326 and/or identification number 328) of the pop-up display panel 550. A location code 558 (e.g., location code 324) indicating where the patient is being treated may also be included in the pop-up display panel 550. The additional information may be displayed in one or more informational rows 560, and may include an identification of the criterion (e.g., abnormal blood pressure) and one or more representative blood pressure measurements (e.g., two measurements separated by approximately 24 hours). Similar to pop-up display panel 500, pop-up display panel 550 may include any escalation and/or general comments entered in by a care provider, as well as any other relevant/desired information, and may include text entry boxes to allow a user to enter new escalation or general comments. In some embodiments, pop-up display panel 550 may include additional controls, such as buttons that when selected, may display different or additional information, and/or may generate additional display panels.

In another example, FIG. 7 shows an example pop-up display panel 700 of the UI 300, which may be displayed in response to the user selecting a downgrade recommendation on the display screen with a second type of user input. Pop-up display panel 700 may include additional information relating to the downgrade recommendation of the patient, who may be referenced by a name 704 and/or an identification number 706 (e.g., patient name 326 and/or identification number 328) of the pop-up display panel 700. The downgrade recommendation for that patient may be included on the pop-up display panel 700 as a downgrade recommendation button 708, herein indicating that the patient is not eligible for a downgrade. The criteria leading to the downgrade recommendation may be displayed in the pop-up display panel 700 and may be organized into two categories, a "Criteria Met" category 710 and an "Additional Criteria Considered" category 712.

In each category, the specific criteria considered for that patient may be displayed with more information than on the UI 300. For example, in the "Criteria Met" category 710, the criteria that led to the downgrade recommendation are listed along with a category of the criteria and a description of the criteria. In the example shown, the downgrade recommendation of "Not eligible" was based on the patient having an abnormal heart rate and ventilator status, and the additional information (e.g., the description) of each criterion in the category 710 includes a description of that criterion, such as the abnormal heart rate including the patient's heart rate being less than 50 or greater than 120 and the ventilator status including the patient being on invasive ventilation.

Similar information may be provided for the category 712, such that a description of each additional criterion that was considered for the recommendation is displayed. In some embodiments, pop-up display panel 700 may include additional controls, such as buttons that when selected, may display different or additional information, and/or may generate additional display panels. For example, a user input selecting one of the displayed criteria (e.g., the abnormal heart rate) may result in measured heart rate values for the patient being displayed. In such an example, the selection of the criterion may cause the EHR of the patient to be launched so that the data in the EHR can be reached directly from the UI 300 and/or pop-up display panel 700.In this way, an efficient presentation is provided to the user as to the various criteria and how they are affecting the current recommendation so that the user can visualize what is needed to achieve a successful downgrade recommendation. By enabling the user to navigate to this easily, while also providing a summary, a more effective user interface is achieved.

Returning to FIG. 3, the downgrade recommendations 336 displayed in UI 300 may be accompanied by other visual elements or visual controls relevant to confirming or rejecting a downgrade recommendation, such as acuity scores. For example, a Glasgow Coma Scale (GCS) rating 340 may be included, which may describe a level of consciousness of the patient, as well as a modified early warning sign (MEWS) score 338, which may be used to identify trends in the patient's condition. The GCS rating 340 and/or the MEWS score 338 may be color-coded to indicate whether the GCS rating 340 and/or the MEWS score 338 are within threshold ranges. For example, if a patient has a GCS rating 340 that is within a first threshold GCS rating and a second threshold GCS rating, the GCS rating 340 may be a first color; if the patient has a GCS rating 340 that is below the first threshold GCS rating, the GCS rating 340 may be a second color; and if the patient has a GCS rating 340 that is above the second threshold GCS rating, the GCS rating 340 may be a third color. In some embodiments, the user may select the GCS rating 340 and the MEWS score 338 to display additional information related to the GCS rating 340 and the MEWS score 338.

After the downgrade recommendations 336 have been generated and displayed on the screen, a care provider may confirm a downgrade recommendation 336, for example, to initiate a process for transferring the patient to a different care unit (e.g., a floor unit) of a hospital. In some embodiments, the care provider may confirm the downgrade recommendation 336 by selecting a control displayed in a row corresponding to the patient in UI 300 (not shown in FIG. 3). For example, in some embodiments, a confirm button may be included in the row, or the confirm button may be included on a pop-up display panel (e.g., the pop-up display panel 500 of FIG. 5A or pop-up display panel 700 of FIG. 7) that may be displayed when the care provider selects the downgrade recommendation 336.

When a downgrade recommendation 336 is confirmed, a transfer order may be created for the patient. When the transfer order is created, the downgrade recommendation 336 may be replaced by a transfer status element 348. The transfer status element 348 may indicate, for example, that an active transfer order exists, whether a receiving unit has been identified or selected, whether the patient has been assigned, whether one or more steps in a transfer process have been carried out, and so forth. If a receiving unit has been assigned, a service line, unit, or bed may be indicated (e.g., based on a highest level of specificity). In some embodiments, when a transfer order is created, one or more downgrade readiness icons may be included in the icons 330 of the patient data panel 332. The one or more downgrade readiness icons may indicate a readiness of the patient to be transferred to a different care unit. The readiness icons may indicate, for example, the different care unit where the patient will be received, or an availability of one or more resources (e.g., beds, etc.) at the different care unit or other information. In this way, a notification may be received that a transfer order has been received for a selected patient and, in response, a downgrade recommendation on the recommendation GUI may be updated for the selected patient to indicate the transfer order.

Referring briefly to FIG. 6, an exemplary set of readiness indicators 600 is shown that may be displayed for a patient awaiting a transfer. The set of readiness indicators 600 may include, for example, a readiness indicator 602, which may indicate that a resource (e.g., a bed) is assigned and clean beyond a threshold; a readiness indicator 604, which may indicate that the bed is assigned and clean; a readiness indicator 606, which may indicate that the bed is assigned and dirty; and a readiness indicator 608, which may indicate that the bed is assigned but occupied by another patient. Additionally, an unassigned transfer order indicator 610 may be displayed in a patient row in place of a downgrade recommendation, which may indicate that a receiving unit has been requested but a transfer order has not yet been assigned or an unassigned transfer order indicator 612, which may indicate that that a receiving unit has not been requested and a transfer order has not yet been assigned.

Returning to FIG. 3, UI 300 may include a milestones, tasks, and alerts column 322, which may show one or more discharge milestones pending completion, patient alerts, and/or contributing factors to a downgrade recommendation. In some embodiments, one or more alert elements 344 may be included in a row of UI 300 corresponding to a patient. The one or more alert elements 344 may include patient alerts generated by one or more EHR systems or other computer systems of a healthcare organization of which the ICU is a part. For example, an EHR system may generate a flag for a patient indicating that the patient is overdue for a test or examination, that certain records or paperwork are missing, that a patient is scheduled for a procedure but has lab results that may put the patient at risk during the procedure, or other suitable alert. The one or more alert elements 344 may also include one or more pending discharge milestones 342, representing tasks to be carried out or completed prior to downgrading the patient or releasing a downgraded patient, pending reports or results, operational logistics involved in transferring the patient, and/or other reminders, warnings, or additional information. In some embodiments, the one or more alert elements 344 may be controls that may be selected. When an alert element is selected, additional information regarding the alert element may be displayed via an additional (e.g., pop-up) display panel, or an external system (such as an EHR system) may be launched to allow a user to view the additional information.

The milestones, tasks, and alerts column 322 may include one or more downgrade alerts 346 (shown in FIG. 3 as rounded rectangles). If a patient is assigned a downgrade recommendation indicating that the patient is not or may not be eligible for transfer to a different care unit, one or more downgrade criteria that were not satisfied when generating the downgrade recommendation (or one or more downgrade criteria that contributed to the downgrade recommendation, which may include criteria being met, such as the abnormal heart rate or ventilation status described above) may be displayed in the one or more downgrade alerts 346. Each downgrade criteria of the one or more downgrade criteria may correspond to a computerized disqualification rule that was applied to patient data relating to the downgrade criteria, where a condition imposed by the rule was not met (or not met, depending on the criteria) by the patient data. By viewing the one or more downgrade criteria elements that contributed to the downgrade recommendation, a care provider at the ICU may quickly and efficiently determine why the patient is ineligible for a downgrade and/or what contributing factors would have to change in order for the patient to be downgraded. Specifically, when estimating a future availability of resources of a plurality of ICUs and/or a hospital system, a large number of patients may be displayed in UI 300. By displaying the downgrade alerts 346 in a compact view in each patient row, an amount of time spent by the care provider scanning through the patients to estimate the future availability may be reduced when compared to accessing patient data via one or more EHR systems.

For example, the patient shown in FIG. 3 in the second row of UI 300 has received a downgrade recommendation of "Not Eligible". Under the downgrade readiness column 320, three downgrade alerts 346 are displayed indicating downgrade criteria that may have been of particular importance in generating the recommendation: a first downgrade criterion indicating that the patient has a central line to the aorta, a second downgrade criterion indicating that the patient suffers from an abnormal respiratory rate, and a third downgrade criterion indicating that the patient is currently on vasoactive medications. This may indicate that the downgrade recommendation of "Not Eligible" was generated for the patient based on a first rule of the patient downgrade recommendation system, where the first rule imposed a condition that patients with a central line shall be treated in the ICU; and based on a second rule of the patient downgrade recommendation system, where the second rule imposed a condition that patients with abnormal respiratory rates shall be treated in the ICU; and based on a third rule of the patient downgrade recommendation system, where the third rule imposed a condition that patients on vasoactive medications shall be treated in the ICU. Generation of the downgrade recommendations in accordance with rules and downgrade criteria is described in greater detail below in reference to FIGS. 4A and 4B.

As described above in reference to the downgrade recommendation 336, in some embodiments, the downgrade alert 346 may be a selectable control (e.g., a button) that when selected may display additional information on the downgrade criterion associated with the downgrade alert 346. In other embodiments, selectable controls may be included within the downgrade alert 346. For example, a comment button may be included (not shown in FIG. 3), which when selected may pop up a display panel where a comment relating to the criterion associated with the downgrade alert 346 may be read, written, replied to, or deleted. A warning flag may also be included, which when selected may pop up a display panel with a textual warning message.

In some embodiments, a shape, texture, and/or color of the downgrade alert 346 may be used to distinguish the downgrade alert 346 from a patient alert (e.g., the one or more alert elements 344) or a discharge milestone 342. For example, the downgrade alert 346 may have an outline with rounded corners, while a patient alert may have an outline with squared corners.

In some embodiments, the patient downgrade recommendation system may be launched by a care provider when the care provider wishes to determine if one or more patients of an ICU may be downgraded and transferred. In other embodiments, the patient downgrade recommendation system may be continuously running, and elements of the patient downgrade recommendation system and/or patient data may be periodically updated automatically by the patient downgrade recommendation system. For example, a care provider may start the patient downgrade recommendation system and view downgrade recommendations of a plurality of patients at a first time. At the first time, the care provider may see that no patients are eligible for a downgrade. The care provider may leave the patient downgrade recommendation system to attend to patients, and return later to the patient downgrade recommendation system, which may still be running, to view the downgrade recommendations of the plurality of patients at a second time. In between the first time and the second time, elements of patient data and/or downgrade recommendations may change, for example, due to more recent data being recorded in one or more EHR systems. As a result of the more recent data being recorded, at the second time, the care provider may see that a patient that was previously not eligible for a downgrade is now eligible for a downgrade. Thus, the patient downgrade recommendation system may serve to provide continuously updated data that may aid the care provider and managing patients of the ICU.

Additionally, data retrieved, accessed, or used by the patient downgrade recommendation system may become temporarily or periodically unavailable, for example, if a system or network coupled to the patient downgrade recommendation system experiences a failure. In the event that data is unavailable at a time when a care provider is requesting downgrade recommendations from the patient downgrade recommendation system, the patient downgrade recommendation system may provide an indication of the unavailability of the data in the UI 300. For example, a downgrade recommendation 336 may be replaced with a different graphical element indicating that a corresponding downgrade recommendation is pending the availability of the data (e.g., restoration of the system/network that failed).

For example, in order to apply a rule to determine whether a blood pressure of the patient is within a suitable range to permit a transfer, the patient downgrade recommendation system may attempt to retrieve a recent blood pressure result from one or more EHRs coupled to the patient downgrade recommendation system. A most recent blood pressure result retrieved from the one or more EHRs may not meet a threshold date or time for being sufficiently recent. In response to the most recent blood pressure result not being sufficiently recent, the patient downgrade recommendation system may not display a downgrade recommendation for the patient, and may display an alternative element indicating that a sufficiently recent blood pressure reading is unavailable. In some embodiments, the most recent blood pressure result may be indicated along with a date and/or time of the most recent blood pressure result.

Thus, while patient data relevant to the downgrade is updated asynchronously in one or more systems and/or databases coupled to the patient downgrade recommendation system, a most recent version of the patient data may be consistently updated and displayed in real time in the UI 300. In this way, the patient downgrade recommendation system may provide an efficient and user-friendly way to monitor patient data relating to a downgrade via a single interface, without having to repeatedly access various EHR systems to ensure accuracy of the patient data and/or while the various EHR systems are in an unlaunched state (e.g., not consuming computing and/or network resources of the healthcare system). An additional advantage of using the patient downgrade recommendation system is that errors made inappropriately downgrading a patient due to patient data that is out of date may be reduced. If the care provider wishes to verify an element of patient data displayed in the UI 300, the care provider may launch a relevant EHR system to verify the element from the patient downgrade recommendation system (e.g., by selecting a downgrade recommendation 336, an alert element 344, a discharge milestone 342, downgrade alert 346, or a different control of the UI 300).

Alternatively, in various embodiments, the care provider may open the patient downgrade recommendation system by first logging into a patient management system of a healthcare network, and selecting a patient downgrade recommendation system icon, tile, or similar menu listing of one or more options for retrieving patient data of the patient management system to start the patient downgrade recommendation system. In still other embodiments, the patient downgrade recommendation system may be launched from within a different computer system of a hospital, such as an EHR system, where the patient downgrade recommendation system may be listed as a selectable menu option within a UI of the EHR system.

As an example of how UI 300 may be used, a care provider may oversee an ICU of a hospital with 10 patients, where all beds of the ICU are occupied. In preparation for two new patients, the care provider may wish to transfer two of the 10 patients to other units of the hospital. In order to transfer two patients out of the ICU, the care provider may wish to determine which of the 10 current patients may be most suitable for downgrading, a prior condition for a transfer.

The care provider may start a computer application running the patient downgrade recommendation system in one of the ways described above, upon which the UI 300 may appear on a display device. The care provider may select the ICU from hospital unit selection element 314. The patient downgrade recommendation system may retrieve a list of patients at the ICU from a separate computer system of the ICU, and generate a plurality of downgrade recommendations for the patients listed in the ICU. When downgrade recommendations have been generated by the patient downgrade recommendation system for all of the patients on the list of patients, the downgrade recommendations may be outputted to the UI 300 for display. Predefined visual elements/controls that correspond to the downgrade recommendations (e.g., the downgrade recommendation elements 336) may be selected and inserted into a template of the UI 300 along with corresponding patient identifying data. Additionally, patient alert, discharge milestone, and downgrade alert elements may be generated (based, for example, on rules that failed) and displayed in relevant patient rows of the UI 300.

When the UI 300 displays the downgrade recommendations generated by the patient downgrade recommendation system, the care provider may see that two of the 10 patients of the ICU have received downgrade recommendations indicating that the patients are ready to downgrade, which may be displayed in a descending order according to readiness. The care provider may select a downgrade recommendation 336 of a patient at the top of the list (e.g., most ready) to pop up an additional display panel (e.g., pop-up display panel 500 or pop-up display panel 700) with more information about the downgrade recommendation. The care provider may review the information, and confirm the downgrade recommendation by selecting a "confirm" button on the additional display panel or enter a downgrade order via a separate system. When the downgrade recommendation is confirmed, the care provider may be prompted to initiate a request to transfer the patient by searching for and/or selecting a receiving care unit, or the request to transfer the patient may be automatically initiated.

Further, via the UI 300, the care provider may view one or more alerts generated for the patient, and initiate any tasks that may be entailed by a transfer order. As the provider completes a task or meets a milestone relevant to the transfer order, the provider may dismiss one or more of the one or more alerts, for example, by selecting a control in a pop-up display panel triggered by selecting an alert. The care provider may then confirm the second patient on the list (in order of readiness) in the same manner. Before closing the application, the care provider may review the downgrade recommendations of other patients on the list whose eligibility for downgrading was not recommended. The care provider may see that a third patient may be eligible for a step-down, intermediate care unit, and click on a downgrade criteria element indicating that the third patient is on a non-invasive ventilator. The care provider may search for an appropriate step-down unit, and discover that the appropriate step-down unit has an opening scheduled for the following week. The care provider may select a comment control of the downgrade criteria element, and record the opening in a comment for other care providers of the ICU.

In some examples, once the downgrade recommendation system determines that a patient is eligible for a downgrade and outputs the downgrade recommendation, a provider may confirm that the patient is ready for a downgrade and initiate a transfer of the patient out of the current hospital unit (e.g., ICU). Once the patient is actually moved to a different unit, the downgrade recommendation system may receive an updated location of the patient. Once the patient location is updated, the patient is removed from UI 300 for the current hospital unit (e.g., the ICU). If the patient is moved to a different location that is still monitored by the downgrade recommendation system, the patient will be monitored in a different location for different downgrades with different criteria.

Referring now to FIG. 4A, a method 400 is shown for generating patient downgrade recommendations for a selected plurality of patients, according to an embodiment of the disclosure. Method 400 may be carried out by a patient downgrade recommendation system, such as the patient downgrade recommendation system 220 of FIG. 2, according to instructions stored in a memory (e.g., memory 204) of the patient downgrade recommendation system, which may be executed by a processor (e.g., processor(s) 206) of the patient downgrade recommendation system.

Method 400 begins at 402, where method 400 includes receiving a request to generate patient downgrade recommendations (also referred to herein as a downgrade request). In various embodiments, the downgrade request may be initiated automatically by the downgrade recommendation system, such that downgrade recommendations are generated for all eligible patients on a regular basis (e.g., each minute). In some examples, the downgrade request may be initiated by a care provider or other user. In either example, the patient downgrade recommendations may be generated for patients of an ICU or similar specialty care unit of a healthcare organization or network (e.g., a hospital), where the ICU or similar specialty care unit has greater or specific resources that may be unavailable at other care units. To initiate the downgrade request, the care provider may open a patient downgrade recommendation system application running on a computer or network terminal of the ICU and select one or more suitable controls of a UI (e.g., UI 300) of the patient downgrade recommendation system (e.g., a clinician may select a user interface button displayed on a display device to request patient downgrade recommendations to be displayed). In some embodiments, the care provider may initiate a downgrade request as part of a routine procedure, (e.g., a daily procedure) in accordance with one or more clinical guidelines of the healthcare organization, as part of routine patient management practices. In other embodiments, the care provider may initiate a downgrade request in response to a need for resources of the ICU. For example, the need for resources of the ICU may result from an epidemic or pandemic, or a mass casualty event, upon which an urgent need for beds for new patients arises.

At 404, method 400 includes receiving a selection of patients to analyze in order to generate the downgrade recommendations. In some embodiments, the patient downgrade recommendation system may be hosted on a server (e.g., of the healthcare organization) and accessible to a plurality of ICUs via a network, and the care provider may enter in a name of the ICU or select the ICU from a list of ICUs in order to indicate the selection of patients. In other embodiments, a relevant ICU may automatically be selected based on a pre-defined configuration file created during a setup stage of the patient downgrade recommendation system.

The selection of patients may be a total number of patients of the ICU, or a subset of the patients of the ICU. In other words, to reduce a duration of processing and receive results more quickly, the care provider may select a small group of patients of the ICU to quickly determine which patients of the small group of patients may be ready to be downgraded, rather than wait for results from a larger group of patients. For example, the care provider may reduce a number of patients to request downgrade recommendations for by using one or more of the filters described in reference to FIG. 3. In other examples, a downgrade recommendation may be generated for each patient in the hospital unit and only a selected set of the downgrade recommendations may be displayed via the user interface, based on the filtering/user selections discussed above.

At 406, method 400 includes generating a downgrade recommendation for each patient of the selection of patients. Generating the downgrade recommendation for each patient may include consulting clinical information of the patient and submitting the information to a patient downgrade recommendation module, as described in greater detail below in reference to FIG. 4B.

At 408, method 400 includes displaying the downgrade recommendations of the selected patients on the display screen of the patient downgrade recommendation system. In various embodiments, the downgrade recommendations may be drawn from a list of possible downgrade recommendations, where each patient of the selection of patients may be assigned a downgrade recommendation from the list of possible downgrade recommendations. The downgrade recommendations may be presented on the screen in a UI of the patient downgrade recommendation system, for example, as described in reference to FIG. 3 above.

The patients may be displayed in a list on the screen in a predetermined order, based on downgrade criteria and/or guidelines of the ICU. In some embodiments, the downgrade recommendation of a patient may be displayed in a row (or column) corresponding to the patient and identified by a patient identifier (e.g., a name, ID, etc.). In other embodiments, displaying the downgrade recommendations of the selected patients on the display screen may include ranking the patients displayed on the display screen by downgrade recommendation (e.g., displayed in a different order for ease of use). For example, one downgrade recommendation may comprise the patient being eligible for a downgrade to a different medical unit, another downgrade recommendation may comprise the patient being potentially eligible for a downgrade, depending on need for ICU resources, and yet another downgrade recommendation may be that the patient is not eligible for downgrade (e.g., because the patient is currently relying on a resource of the ICU). In some embodiments, the patient downgrade recommendation system may display patients that are eligible for a downgrade first (e.g., at a top of a list of downgrade recommendations displayed on the display screen), followed by patients who may be eligible for a downgrade, and with patients not eligible for downgrade towards or at the bottom of the list. In this way, a care provider may view at a glance a number of patients that may be downgraded to free up resources of the ICU, and which patients may be downgraded.

Further, at 410, one or more downgrade elements associated with one or more downgrade recommendations may be displayed. As explained above with respect to FIG. 3, the downgrade elements (such as downgrade alerts 346) may indicate the criteria that contributed to the associated downgrade recommendation. Thus, for one or more or each patient, one or more downgrade elements may be displayed in the user interface.

At 412, method 400 determines if a user has selected a downgrade recommendation or a downgrade element. The user (which may be a care provider) may enter a user input to a downgrade recommendation button or a downgrade element, such as a mouse or touch hover, or a mouse click or touch input, in order to view additional information associated with the downgrade recommendation. If the user has selected a downgrade recommendation or element, method 400 proceeds to 414 to display additional information based on the input. Displaying the additional information may include, as indicated at 416, displaying a pop-up display based on the user input, such as pop-up display panel 500 or pop-up display panel 700. As explained previously, a first pop-up display (e.g., pop-up display panel 500) may be displayed in response to a first user input (e.g., a hover) and a second pop-up display (e.g., pop-up display panel 700) may be displayed in response to a second user input (e.g., a touch input). In some examples, displaying the additional information may include, as indicated at 418, displaying information related to selected downgrade recommendation criteria. For example, when a user selects a downgrade element for a patient, information relating to that downgrade element may be displayed. Because the downgrade elements display criteria that contributed to a downgrade recommendation for that patient, patient-specific information about that criterion may be displayed, such as prior values for that criterion, where available (e.g., patient heart rate over the past 24 hours). The patient-specific information may be obtained from the patient's EHR. In some examples, the user input may cause the EHR to launch and the additional information may be displayed directly from the EHR

If the user does not select a downgrade recommendation or downgrade element, or following the display of the additional information, method 400 optionally includes receiving confirmations and/or adjustments of one or more downgrade recommendations from the user. In various embodiments, the user may be prompted and/or permitted to confirm downgrade recommendations. For example, a visual element comprising a downgrade recommendation in the UI of the patient downgrade recommendation system may be selectable, where selecting the visual element may result in a dialog box prompting the user to confirm the downgrade recommendation, or a separate control element (e.g., a button, toggle switch, etc.) may be provided for confirming the downgrade recommendation. In some embodiments, when a downgrade recommendation is confirmed, a downgrade procedure of the ICU to transfer the patient to a different care unit may be initiated. For example, as indicated at 422, method 400 optionally includes initiating a transfer order, which may be performed in response to the confirmation of the downgrade recommendation (when the downgrade recommendation includes the patient being eligible for a downgrade). Initiating the transfer order may include entering the transfer order into a computerized provider order entry (CPOE) system, which may be executed as part of the EHR system or executed as a separate system.

In some circumstances, the care provider may not confirm the downgrade recommendation, and the care provider may adjust the downgrade recommendation. For example, the patient downgrade recommendation system may generate a downgrade recommendation for a patient indicating that the patient may be potentially eligible for a downgrade and a transfer to a different care unit of a hospital. However, the care provider may determine, for example, based on a history or additional clinical information of the patient, based on operational information of the healthcare organization, or based on other information, that the patient should not be eligible for a downgrade. As a result of the care provider deciding that the patient should not be eligible for a downgrade, the care provider may change the downgrade recommendation from potentially eligible for a downgrade to not eligible for a downgrade. Specifically, the care provider may select a control element (e.g., a downgrade recommendation element 336) of the UI of the patient downgrade recommendation system to generate an additional display panel (e.g., the pop-up display panel 500), and alter the downgrade recommendation via one or more controls of the additional display panel. By adjusting the downgrade recommendation from potentially eligible for a downgrade to not eligible for a downgrade, other care providers of the ICU and/or the healthcare organization may not consider the patient potentially eligible for a downgrade. If the user enters an adjustment to a downgrade recommendation, the recommendation may be adjusted, as indicated at 422, which may include saving the adjustment in memory and transmitting the adjusted status to other devices displaying the UI. Method 400 then ends.

Referring now to FIG. 4B, a method 450 for generating a patient downgrade recommendation for an individual patient is shown, according to an embodiment of the disclosure. Method 450 may be carried out by a patient downgrade recommendation module of a patient downgrade recommendation system, such as patient downgrade recommendation module 240 of downgrade recommendation system 220, according to instructions stored in memory (e.g., the memory 204), which may be executed by a processor (e.g., processor(s) 206) of the downgrade recommendation system.

Method 450 begins at 452, where method 400 includes identifying a patient for which a downgrade recommendation is to be made. The patient downgrade recommendation system may iteratively generate and update downgrade recommendations for each patient in a given hospital unit, such as an ICU. When the patient downgrade recommendation system generates a downgrade recommendation for a given patient, the patient downgrade recommendation system may input an identifier of that patient into the patient downgrade recommendation module, to receive, as an output of the patient downgrade recommendation module, a downgrade recommendation for the relevant patient.

At 454, method 450 includes identifying a set of downgrade criteria relevant to a downgrade recommendation for the patient. In some embodiments, the set of downgrade criteria may be based, for example, on an ICU type in which the patient is currently located. As there may be different types of ICUs within the healthcare organization, each ICU may include different downgrade criteria that may affect what patient data is analyzed to determine the downgrade recommendation. For example, a first set of patient data may be obtained and analyzed to determine whether an adult patient may be released from a first ICU, and a second set of patient data may be obtained and analyzed to determine whether a baby may be released from a neonatal ICU.

At 456, method 450 includes applying an appropriate set of disqualification rules to the patient data corresponding to the selected set of downgrade criteria, to generate the downgrade recommendation. Specifically, each rule of the set of disqualification rules may be associated with a corresponding criterion of the downgrade criteria. For example, if it is determined that the patient is located in a first ICU type, a first set of disqualification rules based on a first set of downgrade criteria may be applied to the patient data to determine whether the patient is eligible for a downgrade, while if the patient is located in a second ICU type, a second set of disqualification rules based on a second set of downgrade criteria may be applied to the patient data.

The disqualification rules may be stored in a database, where each disqualification role of the disqualification rules may be included in a separate record in the database. Thus, when applying the disqualification rules, the patient downgrade recommendation module may determine which disqualification rules are to be applied, based on the downgrade criteria, and retrieve each disqualification rule of the disqualification rules to be applied individually from the database. Thus, a customized set of disqualification rules for a given patient may be quickly and efficiently assembled and applied.

At 458, method 400 includes retrieving patient data for the downgrade criteria relevant to the downgrade recommendation. Once the patient downgrade recommendation module has determined the type of ICU in which the patient is being treated, the patient downgrade recommendation module may proceed to retrieve patient data for the relevant downgrade criteria. In various embodiments, the patient data for the relevant downgrade criteria may be retrieved from one or more EHR and/or other computer systems of the ICU and/or the healthcare organization. The other computer systems may include a local monitoring application of the ICU used to make routine measurements (e.g., current vital signs) or tests of the patient. In other words, a first set of patient data may be a comprehensive set of data of the patient, and the first set of patient data may be stored across a plurality of computer systems (e.g., EHR systems) and/or databases of a healthcare system. Out of the first set of patient data, a second, smaller set of patient data (e.g., a subset) may be retrieved, where the second, smaller set of patient data is patient data specific to the relevant downgrade criteria.

In some embodiments, vital signs or clinical markers may be retrieved from a single source, such as a single EHR system, where the patient downgrade recommendation module may retrieve a plurality of records of the vital sign or clinical marker and select a most recent vital sign or clinical marker from the plurality of records. Further, the patient downgrade recommendation module may analyze the plurality of records to determine one or more trends in vital signs or clinical markers, where the one or more trends may be used in a disqualification rule. In some examples, the patient downgrade recommendation system may receive a data feed from the EHR that includes the patient parameters (e.g., vital signs, ventilation status, and care provider-entered information such as Glasgow coma score) dictated by the downgrade criteria for the patient. By obtaining the patient parameters from a single source on a semi-continuous feed (e.g., updated every 10, 15, or 30 seconds), the efficiency of the downgrade recommendation system may be improved by reducing processing demands and network traffic associated with obtaining the patient parameters from multiple sources and/or requesting the patient parameters each time a downgrade recommendation is made. In some examples, the patient downgrade recommendation system may map specific feeds for each data source from the EHR using an API, which is performed for each criterion.

Applying the appropriate set of disqualification rules at 456 may include applying each rule of the set of disqualification rules to patient data matching the criterion associated with the rule. In some embodiments, if a rule is not satisfied (e.g., if a condition established by the rule is not met), the patient may be considered ineligible for downgrading (e.g., disqualified). For example, a rule may indicate threshold values for a measurement of a vital sign (e.g., blood pressure), where if the measurement is above or below the indicated threshold values, the patient may not be eligible for downgrading.

The disqualification rules may address a limited number of downgrade criteria. For example, each disqualification rule may be limited to no more than 3 downgrade criteria to be satisfied by patient data. By limiting the number of downgrade criteria associated with the disqualification rules, a larger number of rules may be applied in a shorter amount of time, resulting in faster results and quicker decisions by a care provider. In some embodiments, the disqualification rules may be applied in series, where if a first disqualification rule fails, no further disqualification rules may be applied. In other embodiments, the disqualification rules may be applied in parallel, where a plurality of results of applying the disqualification rules may be collected and compared after applying the disqualification rules to determine what kind of downgrade recommendation to generate. By applying the disqualification rules in series or in parallel, an amount of time spent and/or an amount of computer and/or network resources consumed by the patient downgrade recommendation module to generate the downgrade recommendations may be reduced. In some examples, once a disqualification rule has been applied and results in a specific downgrade recommendation (e.g., the patient not being eligible for a downgrade), that recommendation may be output, and then all remaining rules may still be applied to identify all the criteria that contributed to the patient's status. In this way, the downgrade recommendation may be generated quickly, but each parameter that contributed to the patient's downgrade status may still be identified so that a care provider can determine all the reasons why the patient did not qualify for a downgrade, for example.

For some patient data, not meeting a single condition established by a disqualification rule may be sufficient to declare the patient ineligible for downgrading. For example, if a resource specific to the ICU (e.g., an invasive ventilator) is being used by the patient, the patient may be declared ineligible for downgrading without considering any other described rules. A sample list of disqualification rules is shown below in Table 1, where for a specific disqualification rule downgrade criteria (shown in column 1), a logic (shown in column 2) is applied, and if the result of applying the logic is TRUE, the patient is assigned to an indicated level of care (shown in column 3):

**Table 1**

| **Criteria** | **Logic** | **Level of Care** |
|---|---|---|
| Abnormal BP | BP Vitals outside of appropriate range (90 < Systolic < 180, 50 < Diastolic < 110) *(configurable)* | ICU |
| Abnormal Respiratory Rate (ICU) | Respiratory Rate outside of appropriate range (10 < RR < 30) *(configurable)* | ICU |
| Cardiac / respiratory arrest | Active order for Code Blue | ICU |
| Central Line | Active LDA for Central Line with 8 French or greater | ICU |
| Continuous Dialysis (CRT/PIRRT) | Active Medication for Dialysis with continuous frequency | ICU |
| Excessive Blood Loss (Declining Hemoglobin) | Hemoglobin lab result decreases by at least 25% in last X hours *(configurable)* | StepDown |
| Abnormal Heart Rate > 120 or < 50 | HR Vitals outside of appropriate range (50 < HR < 120) *(configurable)* | ICU |
| Insulin Drip | Active Medication for Insulin with continuous frequency | ICU |
| Invasive Ventilator w/o Trach | Active LDA for Invasive Ventilator w/o Trach | ICU |
| Invasive Ventilator w/o Trach extubated | Extubated LDA for Invasive Ventilator w/o Trach in last 8 hours *(configurable)* | ICU |
| Lactate > 3-4 | Lactic Acid lab result > 3-4 (configurable) | ICU |
| MEWS >= 4 | MEWS Score >=4 | ICU |
| O2, FIO2 (ICU) | O2, FIO2 > 5L | ICU |
| Q1,Q2 Neurovascular checks | Active orders for Neurovascular Checks with Frequency Every 1-2 Hours | ICU |
| Suction more frequently than every 2 hours | Suction documentation with Frequency > Every 2 Hours in last X Hours *(configurable)* | ICU |
| Swan Ganz Catheter | Active LDA for Swan Ganz Catheter | ICU |
| Abnormal Respiratory Rate (Step Down) | Respiratory Rate outside of appropriate range (12 < RR < 28) *(configurable)* | StepDown |
| Invasive w/ Trach | Active LDA for Invasive Ventilator w/ Trach | StepDown |
| Non-Invasive Ventilator | Active LDA for Non-Invasive Ventilator | StepDown |
| O2, FIO2 (Step Down) | O2, FIO2 > 4L and < 5L | StepDown |
| Suction more frequently than every 4 hours | Suction documentation with Frequency > Every 4 Hours in last X Hours *(configurable)* | StepDown |
| Continuous EEG | Active orders for Continuous EEG | Floor |
| Low Oxygen Saturation < 88% | Oxygen Saturation < 88% *(configurable)* | Floor |

For example, in accordance with the first row, if a systolic blood pressure of the patient is outside of a range of 90-180 and a diastolic blood pressure of the patient is outside of a range of 50-110, the patient may be assigned to an ICU level of care, whereby the patient is not eligible for downgrading.

Alternatively, as shown below in Table 2, conditions of the disqualification rules may be applied to patient data in combination. For example, as shown below, if the patient is determined to have a sodium imbalance, the patient may be assigned to a medical unit on a floor of a hospital. If the patient has a low oxygen saturation (e.g., less than 88%) AND the patient has an abnormal respiratory rate (e.g., less than 12 or greater than 28), the patient may be assigned to a medical ICU. However, if the patient has a low oxygen saturation AND a toxic substance present in bloodwork (or is undergoing continuous EEG), without exhibiting an abnormal respiratory rate, the patient may be assigned to a stepdown (e.g., intermediate) care unit. In this way, conditions applying to different patient data may be combined to generate more accurate downgrade recommendations than may be generated by applying the conditions individually.

**Table 2**

| | Toxic Substance Present in Blood Work | Continuous EEG | Oxygen Saturation < 88% | Abnormal Respiratory rate (<12 or >28) | Non-Invasive Ventilator | Invasive w/ Trach | Suction more frequently than every 4 hours |
|---|---|---|---|---|---|---|---|
| Toxic Substance Present in Blood Work | | | | | | | |
| Continuous EEG | Floor | | | | | | |
| Oxygen Saturation < 88% | Stepdown | Stepdown | | | | | |
| Abnormal Respiratory rate | Stepdown | Stepdown | Medical ICU | | | | |
| Non-Invasive Ventilator | Stepdown | Stepdown | Medical ICU | Medical ICU | | | |
| Invasive w/ Trach | Stepdown | Stepdown | Medical ICU | Medical ICU | | | |
| Suction more frequently than every 4 hours | Stepdown | Stepdown | Medical ICU | Stepdown | Medical ICU | Stepdown | |
| Declining Hemoglobin | Stepdown | Stepdown | Medical ICU | Stepdown | Stepdown | Stepdown | Stepdown |

In some embodiments, the rules may be generated based on clinical guidelines issued by the healthcare organization and/or relevant health authorities, in conjunction with expert opinions of experienced care providers of a relevant ICU.

At 460, method 450 includes outputting a downgrade recommendation and relevant patient information for display on a display device. As described above in reference to FIG. 3, if a downgrade recommendation for a patient is that the patient is not eligible for a downgrade, the patient downgrade recommendation system may provide an indication of any disqualification rules that were not satisfied due to a condition imposed by the rule not being met by the patient data. For example, the patient may rely on a resource of the ICU that is not available at other medical units, or the patient may not be stable enough to transfer. If the resource is being used by the patient, thereby preventing the patient from being downgraded, a downgrade alert (e.g., the downgrade alert 346) may be displayed in a UI of the patient downgrade recommendation system.

Thus, for each downgrade recommendation generated for each patient, one or more downgrade alerts indicating unmet downgrade criteria of the downgrade recommendation may be included with the downgrade recommendation, as described above in reference to FIG. 3. As an example, if a patient is determined not to be eligible for a downgrade due to having a blood pressure exceeding a threshold blood pressure, the rule criterion "Abnormal Blood Pressure" (e.g., from Table 1) may be included with the downgrade recommendation as part of the output of the patient downgrade recommendation module to the patient downgrade recommendation system. If more than one rule is not met by the patient data, additional rule downgrade criteria may be included in the output. Alternatively, if a downgrade recommendation for a patient is that the patient is eligible for a downgrade, no additional information and/or downgrade criteria may be included in the output. The patient downgrade recommendation system may receive the rule criterion "Abnormal Blood Pressure" and any additional rule downgrade criteria from the patient downgrade degradation module, and display it in the UI along with the downgrade recommendation.

As an example of how the patient downgrade recommendation module might work, the patient downgrade recommendation module may retrieve a list of patients of the ICU from an administrative database of the hospital. For each patient on the list of patients, the patient downgrade recommendation system may receive patient parameters (e.g., vital signs, acuity scores) from one or more EHR systems and/or databases of the hospital. Based on characteristics of the ICU (e.g., a type of ICU), the patient downgrade recommendation system may select a set of downgrade criteria and corresponding rules which may be applied to determine whether the patient may be downgraded or not. The patient downgrade recommendation system may iterate through the downgrade criteria, and for each downgrade criterion, the patient downgrade recommendation system may retrieve patient data matching or relating to the downgrade criterion. The patient downgrade recommendation system may apply a disqualification rule corresponding to the downgrade criterion to the patient data matching the downgrade criterion, to determine whether a condition imposed by the disqualification rule is met by the patient data or not. For example, if the downgrade criterion is a GCS score, and the disqualification rule indicates that the GCS score must exceed a threshold GCS score for the patient to be eligible for a downgrade, the patient downgrade recommendation system may receive a GCS score of the patient from the EHR and determine whether the GCS score exceeds the threshold GCS score. After applying all the disqualification rules to all the downgrade criteria selected for the patient, the patient downgrade recommendation module may analyze (e.g., apply a logic algorithm to) any disqualification rules that were not met for the patient, and generate an appropriate downgrade recommendation. In some cases, a single rule may disqualify the patient from being eligible for a downgrade, while in other cases, a plurality of rules may be analyzed in conjunction to determine a downgrade recommendation.

FIG. 8 is a flow chart illustrating a method 800 for viewing a recommendation GUI that may be output by a downgrade recommendation system, such as system 220 of FIG. 2. Method 800 may be implemented by one or more aspects of a medical information system, such as medical information system 200, including a downgrade recommendation system and one or more additional computer systems and databases, such as an EHR database. In some examples, aspects of method 800 may be carried out by a patient downgrade recommendation system, such as the patient downgrade recommendation system 220 of FIG. 2, according to instructions stored in a memory (e.g., memory 204) of the patient downgrade recommendation system, which may be executed by a processor (e.g., processor(s) 206) of the patient downgrade recommendation system.

At 802, method 800 includes displaying a menu listing options for retrieving data. The data may include data of one or more patients and may be obtained from a plurality of databases and systems of a hospital. As indicated at 804, the options listed in the menu may include an option to retrieve data from one or more electronic health record (EHR) systems. Further, the options listed in the menu may include an option to display a recommendation graphical user interface (GUI), and thus the menu may include a link to a recommendation GUI, as indicated at 805. In some examples, the recommendation GUI may be reached directly from the one or more EHR systems. For example, a user may launch an EHR interface whereby one or more EHRs of a patient may be viewed. The EHR interface may include a link to the recommendation GUI that, when selected, launches the recommendation GUI. In some examples, the menu may include a command center or hospital systems menu whereby the recommendation GUI and the EHR interface may be reached via separate menu options.

At 806, method 800 includes displaying the recommendation GUI. As explained previously, the recommendation GUI may be displayed in response to selection of a link, which may be displayed via the EHR interface, a command center interface, a hospital systems menu, or other suitable interface. The recommendation GUI may display a respective downgrade recommendation for one or more patients, where each downgrade recommendation recommends whether or not to downgrade the patient, as indicated at 808. The downgrade recommendations may be generated as explained above with respect to FIG. 4B, and the downgrade recommendations may be displayed in the recommendation GUI as user interface elements, such as the downgrade recommendations 336 of FIG. 3.

In some examples, the recommendation GUI may display additional display elements, as indicated at 808. In some examples, a downgrade recommendation may be selectable to add additional display elements to the recommendation GUI and the additional display elements may identify parameters from the retrieved data from the one or more EHR systems that triggered a displayed downgrade recommendation. For example, a downgrade recommendation displayed on the recommendation GUI may be selectable to cause a pop-up display panel, such as pop-up display panel 550 of FIG. 5B or pop-up display panel 700 of FIG. 7, to be displayed. Thus, as indicated at 812, additional display panels may be displayed via the recommendation GUI, in response to selection of a downgrade recommendation or another user interface element. For example, a downgrade recommendation may be selectable to launch a first display panel with additional information and/or controls relating to the associated downgrade recommendation (e.g., pop-up display panel 500 and/or pop-up display panel 700), and the recommendation GUI may further include a limited set of additional visual elements that are selectable to launch a second display panel. The limited set of additional visual elements may include, for each of one or more patients, one or more causal criteria of the downgrade recommendation for that patient, the one or more causal criteria each including a criterion of the plurality of criteria that triggered the downgrade recommendation. For example, the limited set of visual elements may be the downgrade elements/alerts described above with respect to FIG. 3, such as the downgrade alerts 346. If one or more visual elements (e.g., a downgrade element) is selected, a pop-up display panel, such as pop-up display panel 550, may be displayed. In this way, the second display panel may include one or more values of a selected causal criterion, the one or values obtained from the retrieved data (e.g., from the EHRs).

When the recommendation GUI is displayed and/or when the additional display elements (e.g., the limited set of visual elements and/or the various pop-up display panels described herein) are displayed, the one or more EHRs may exist in an un-launched state (e.g., not displayed and not currently accessed by the display device). In this way, the data from the EHRs may be used to generate the downgrade recommendations and values of causal criteria may be obtained from the EHRs (e.g., abnormal heart rate, abnormal respiration rate) and used to populate additional display panels without the user having to access the EHRs themselves (e.g., in a separate window). In doing so, the recommendation GUI as disclosed herein may present a limited set of information (e.g., downgrade recommendations and causal criteria triggering the downgrade recommendations) to the users/care providers in order to increase the efficiency of the users' interaction with the available data, as users are not forced to sift through multiple separate EHRs, data feeds, data files, etc., to identify and then aggregate the needed information.

The systems, methods, and graphical user interfaces provided herein may improve the accuracy and timeliness of patient downgrades and transfers out of high-demand, high-resource hospital units such as ICUs, which may be particularly useful during high-demand situations where time is limited, such as during infectious disease outbreaks where patient demand of resources may change exponentially and data obtained one day may not be applicable the next day. The time it would take to individually collect data from multiple EHRs, aggregate and analyze the collected data, and visualize the data using standard methods could render the data useless by the time the data was visualized, because patient conditions may change so rapidly. By establishing a system that automatically receives data from all EHRs in real time or near real time, aggregates that information regardless of data format, and continually updates a recommendation GUI as data is received, the approach of the disclosure allows care providers to make informed decisions about patient downgrades, such as when to transfer patients and where to transfer the patients, thereby improving patient care.

In contrast, in prior systems when a care provider attempted to determine if a patient was ready for a downgrade/transfer to a different unit, errors could be made due to delays in individual care providers obtaining all necessary parameters for evaluating patient downgrade readiness. For example, a patient's condition may have improved to the point where the patient would be ready for a downgrade to a medical-surgical unit rather than an ICU, but limited care provider resources may result in the patient being kept in the ICU for 12, 24, or more hours than necessary, thereby utilizing ICU resources unnecessarily. The described recommendation GUI solves this problem by providing a specific, dynamically updating list of patient downgrade recommendations and associated causal/triggering criteria. In this way, the recommendation GUI provides an improvement to the capability of the healthcare system as a whole. The disclosure provides a specific way of improving the capability of the healthcare system, by providing one or more recommendation GUIs that display dynamically updating patient downgrade recommendation lists. The disclosure further provides a specific improvement to the way computers operate by aggregating EHR data for multiple patients in one location and updating the patient downgrade recommendations in real-time, which may obviate the need for users to have to navigate through multiple different data files, manually update information as availability changes, and so forth, thereby increasing the efficiency of the operation of the computer for the user.

Further, hospitals may not be configured to share patient downgrade readiness data in real-time, across different units of the hospital. Thus, prior methods of downgrade readiness determination and transfer availability demanded manual collection of data (e.g., reading patient charts in person, discussing patient status with other care providers, visually inspecting each room for bed occupancy) and aggregation in a spreadsheet or other document, with updates also made manually. Systems were not available to automatically pull EHR data from multiple patients in multiple hospital units and then aggregate the pulled data into a visually clear format where healthcare decisions can be quickly made just by glancing at a GUI, as described in the embodiments herein.

The recommendation GUIs described herein provide a specific manner of displaying a limited set of information to a user (patient downgrade readiness information), rather than using conventional user interface methods to display a generic index on a computer, requiring the user to step through many layers of menu options to reach the desired data, or burying the desired data within all hospital data. Thus, the user experience with the computer may be improved and made more efficient.

Furthermore, by displaying a limited set of information via the recommendation GUIs as described herein, operation of the computing device(s) that collect and render the data for display may be improved by reducing the processing demands of the computing device(s), thereby increasing the efficiency of the computing device(s). For example, only certain causal criteria leading to a specific downgrade recommendation may be displayed, which results in a limited amount of the data that is received being processed, which may improve the efficiency of the computing device(s). Further, in some examples the data is processed in real time and updates to the recommendation GUIs are made continuously as data is received, and therefore undue processing lags that may occur if updates were made at predefined discrete time points may be reduced, which may improve the efficiency of the computing device(s). Additionally, as described herein, if network lags or outages result in an incomplete downgrade recommendation (e.g., due to one or more criteria for evaluating downgrade readiness not being currently available), users may be notified that a downgrade recommendation is not available rather than being presented with an incomplete and hence potentially inaccurate downgrade recommendation.

The resource utilization server, included as part of a medical information system as described herein, may provide for high speed data processing that allows for the real-time or near real-time updates to the recommendation GUIs. For example, the patient downgrade recommendation system may be configured to update the recommendation GUIs every 30 seconds, rather than every 15 minutes or longer as in some prior systems.

Thus, via the disclosed patient downgrade recommendation system, recommendations on downgrading a patient and information relevant to a patient status may be displayed in a manner that is easy to visually parse and act on in a reduced amount of time. The patient information may be displayed via small graphical elements with minimal text, which may allow a large number of downgrade recommendations to be included on the same screen. A user may then select a graphical element of interest to view more information about a corresponding event, record, or report. The patient information may be stored in different databases that would otherwise be accessed via individual interfaces, and by using the patient downgrade recommendation system to aggregate the patient information, an amount of time necessary to review relevant patient information for diagnosis and treatment decisions may be reduced. The patient downgrade recommendation system disclosed herein may also aggregate patient data to a single place, into a single application, which helps reduce wasted time spent searching for known but scattered data, and unknown and missing data. This reduces cognitive overloads and aids clinical thinking, because the patient data is reconstructed into a clinically helpful structure. The technical effect of automatically generating downgrade recommendations for an ICU by applying a set of rules to select patient data is that errors due to sorting through large amounts of patient data may be reduced, and an amount of time spent by a clinician making downgrade decisions may be reduced.

In another representation a method for assessing downgrade readiness of patients of an intensive care unit (ICU) via a computing system includes outputting, to a display device of the computing system, a graphical user interface (GUI) that includes, for each patient of a plurality of patients, a downgrade recommendation of the patient and for one or more patients of the plurality of patients, a downgrade alert indicating a downgrade criterion for downgrading the patient that was not met by patient data of the patient; responsive to a first user input associated with a selected recommendation to downgrade the patient, sending an order to initiate a transfer of a patient corresponding to the downgrade recommendation from an ICU to a receiving care unit, and displaying, on the GUI, one or more discharge milestones of the patient; responsive to a second user input associated with a selected recommendation not to downgrade the patient, displaying, on the GUI, a display panel with additional information relating to the selected downgrade recommendation; and responsive to a third user input associated with a selected patient alert, discharge milestone, or downgrade alert, displaying, on the GUI, a display panel with additional information relating to the selected patient alert, discharge milestone, or downgrade alert. In a first example of the method, the patient data that does not meet the downgrade criterion is retrieved from one of a plurality of systems and/or databases coupled to the computing system via a network. In a second example of the method, optionally including the first example, the method further includes automatically updating a patient alert, discharge milestone, downgrade alert, and/or downgrade recommendation in the GUI as additional information becomes available, the additional information retrieved from one of a plurality of systems and/or databases coupled to the computing system via a network.

The disclosure also provides support for a method for a patient downgrade recommendation system of an intensive care unit (ICU), comprising: for each patient of a plurality of selected patients of the ICU: selecting a set of downgrade criteria for the patient, from a first, larger set of patient data of the patient accessible from a plurality of sources communicably coupled to the patient downgrade recommendation system, automatically compiling a second, smaller set of patient data based on the set of downgrade criteria, applying a set of rules associated with the set of downgrade criteria to the second, smaller set of patient data to generate a downgrade recommendation on whether the patient may be downgraded from a first status to a second, lower status, the first status and second status based on one or more medical conditions of the patient, generating a graphical downgrade recommendation element summarizing the downgrade recommendation, arranging each graphical downgrade recommendation element of the plurality of selected patients in a single integrated view of a user interface (UI) of the patient downgrade recommendation system, and displaying the UI on a display device of the patient downgrade recommendation system. In a first example of the method, the first, larger set of patient data of each patient of the plurality of selected patients is distributed across one or more systems and/or databases communicatively coupled to a computer system of the ICU via a network, including one or more Electronic Health Record (EHR) systems. In a second example of the method, optionally including the first example, the method further comprises: in a first condition, in response to the second, smaller set of patient data of a patient meeting conditions imposed by the set of rules, generating a downgrade recommendation that the patient be downgraded, and in a second condition, in response to the second, smaller set of patient data of the patient not meeting one or more of the conditions imposed by the set of rules, generating a downgrade recommendation that the patient not be eligible for a downgrade. In a third example of the method, optionally including one or both of the first and second examples, the method further comprises: in response to clinical information of the patient data being unavailable, the clinical information relied on for applying a rule to the patient data: displaying a textual indication in the UI that a downgrade recommendation of the patient is pending an availability of the clinical information that is unavailable, periodically attempting to retrieve the clinical information, in response to retrieving the clinical information, generating the downgrade recommendation of the patient, and displaying the downgrade recommendation in the UI. In a fourth example of the method, optionally including one or more or each of the first through third examples, arranging the graphical downgrade recommendation elements of the plurality of selected patients in the single integrated view of the UI further comprises displaying, for one or more patients of the plurality of selected patients, a downgrade alert indicating a contributing factor to a downgrade recommendation of the patient. In a fifth example of the method, optionally including one or more or each of the first through fourth examples, arranging the graphical downgrade recommendation elements of the plurality of selected patients in the single integrated view of the UI further comprises displaying, for a patient of the plurality of selected patients, one or more of: an alert generated for the patient by a patient management system coupled to the patient downgrade recommendation system via the network, and a discharge milestone indicating a task pending completion for discharging the patient from the ICU. In a sixth example of the method, optionally including one or more or each of the first through fifth examples, the method further comprises: structuring the set of rules associated with the set of downgrade criteria as a limited list of machine-implementable disqualification rules, each disqualification rule including a condition corresponding to no more than 3 downgrade criteria to be satisfied by patient data of the second, smaller set of patient data, and in response to one or more conditions not being satisfied, disqualifying the patient from being eligible for a downgrade. In a seventh example of the method, optionally including one or more or each of the first through sixth examples, each disqualification rule is stored as a separate record in a database, and applying the set of rules includes: retrieving disqualification rules corresponding to the set of downgrade criteria from the database, applying the disqualification rules in parallel or in series to corresponding data of the second, smaller set of patient data, and generating a downgrade recommendation based on whether the patient was disqualified by one or more of the disqualification rules. In an eighth example of the method, optionally including one or more or each of the first through seventh examples, the second, smaller set of patient data is updated asynchronously, and the patient downgrade recommendation system periodically reapplies the disqualification rules to the second, smaller set of patient data to generate real-time updates to downgrade recommendations. In a ninth example of the method, optionally including one or more or each of the first through eighth examples, the method further comprises: in response to a user selecting a selected graphical downgrade recommendation element of the UI of the patient downgrade recommendation system, generating a first display panel with information of a downgrade recommendation and/or controls associated with the selected graphical downgrade recommendation element, and in response to the user selecting a patient alert, discharge milestone, or downgrade alert of the UI, generating a second display panel with information and/or controls associated with the patient alert, discharge milestone, or downgrade alert. In a tenth example of the method, optionally including one or more or each of the first through ninth examples, the method further comprises: in response to the user of the patient downgrade recommendation system confirming a downgrade recommendation of a patient via a control of the first display panel: initiating a transfer of the patient from the ICU to a receiving care unit, displaying one or more discharge milestones and/or alerts for the patient in the UI, displaying one or more downgrade readiness icons in the UI, indicating a readiness of the patient to be transferred, periodically retrieving updated information of the one or more discharge milestones and/or alerts from one or more systems and/or databases communicatively coupled to the patient downgrade recommendation system via a network, and automatically updating the one or more discharge milestones and/or alerts in the UI with the updated information. In a eleventh example of the method, optionally including one or more or each of the first through tenth examples, the graphical downgrade recommendation element includes one of: an indication that the patient is ready for a transfer, an indication that the patient is ready to downgrade to an intermediate/step-down unit, an indication that the patient may possibly be ready to be downgraded, an indication that the patient has met sub-specialty criteria and is ready to laterally transfer to another ICU, an indication that the patient is not eligible for a downgrade. In a twelfth example of the method, optionally including one or more or each of the first through eleventh examples, automatically compiling the second, smaller set of patient data based on the set of downgrade criteria further includes retrieving a plurality of clinical markers of the patient, each clinical marker of the plurality of clinical markers recorded at a different time, and including in the second, smaller set of patient data one of: a most recent clinical marker of the plurality of clinical markers, and a result of analyzing a trend in the plurality of clinical markers.

The disclosure also provides support for a method for a patient downgrade recommendation system, comprising: receiving, at the patient downgrade recommendation system, a feed from an electronic health record database, where one or more values for each of a plurality of parameters for each of a plurality of patients are sent over the feed, responsive to a first user input selecting a first hospital unit, displaying a recommendation graphical user interface (GUI) that displays, for each patient of a first subset of the plurality of patients, a first downgrade recommendation recommending whether or not to downgrade the patient, where each first downgrade recommendation is generated by applying a first set of downgrade rules to a first set of patient data obtained from the feed, each of the first set of downgrade rules and the first set of patient data selected based on the first hospital unit, and responsive to a second user input selecting a second hospital unit, displaying the recommendation GUI with a second downgrade recommendation for each of a second subset of the plurality of patients, where each second downgrade recommendation is generated by applying a second set of downgrade rules to a second set of patient data obtained from the feed, each of the second set of downgrade rules and the second set of patient data selected based on the second hospital unit. In a first example of the method, the recommendation GUI further comprises, for one or more patients of the first subset of patients, a downgrade alert indicating a contributing factor to a downgrade recommendation of the patient. In a second example of the method, optionally including the first example, each downgrade recommendation includes one of: an indication that the patient is ready for a transfer, an indication that the patient is ready to downgrade to an intermediate/step-down unit, an indication that the patient is possibly ready to be downgraded, an indication that the patient has met sub-specialty criteria and is ready to laterally transfer to another ICU, and an indication that the patient is not eligible for a downgrade.

The disclosure also provides support for a patient downgrade recommendation system, comprising: one or more processors, and memory storing instructions executable by the one or more processors to: receive, at the patient downgrade recommendation system, a feed from an electronic health record database, where one or more values for each of a plurality of parameters for each of a plurality of patients are sent over the feed, output, for display on a display device, a recommendation graphical user interface (GUI) that includes, for one or more patients of the plurality of patients, a downgrade recommendation recommending whether or not to downgrade the patient, where each downgrade recommendation is generated by applying a set of downgrade rules to a set of patient data obtained from the feed, and display, on the recommendation GUI, for a selected patient of the one or more patients, a downgrade element indicating a contributing factor to a downgrade recommendation of the selected patient. In a first example of the system, updated values for each of the plurality of parameters are received in real-time via the feed and wherein each downgrade recommendation is updated at a predetermined frequency. In a second example of the system, optionally including the first example, the instructions are further executable to receive a notification that a transfer order has been received for a selected patient and, in response, update the downgrade recommendation on the recommendation GUI for the selected patient to indicate the transfer order. In a third example of the system, optionally including one or both of the first and second examples, the instructions are further executable to: in response to a first user input to the recommendation GUI, output, for display on the recommendation GUI, a first display panel including a list of all criteria considered in generating a selected downgrade recommendation, and in response to a second user input to the recommendation GUI, output, for display on the recommendation GUI, a second display panel including additional information relating to a selected criterion considered in generating a selected downgrade recommendation.

As used herein, an element or step recited in the singular and proceeded with the word "a" or "an" should be understood as not excluding plural of said elements or steps, unless such exclusion is explicitly stated. Furthermore, references to "one embodiment" of the present invention are not intended to be interpreted as excluding the existence of additional embodiments that also incorporate the recited features. Moreover, unless explicitly stated to the contrary, embodiments "comprising," "including," or "having" an element or a plurality of elements having a particular property may include additional such elements not having that property. The terms "including" and "in which" are used as the plain-language equivalents of the respective terms "comprising" and "wherein." Moreover, the terms "first," "second," and "third," etc. are used merely as labels, and are not intended to impose numerical requirements or a particular positional order on their objects.

This written description uses examples to disclose the invention, including the best mode, and also to enable a person of ordinary skill in the relevant art to practice the invention, including making and using any devices or systems and performing any incorporated methods. The patentable scope of the invention is defined by the claims, and may include other examples that occur to those of ordinary skill in the art. Such other examples are intended to be within the scope of the claims if they have structural elements that do not differ from the literal language of the claims, or if they include equivalent structural elements with insubstantial differences from the literal languages of the claims.

## Claims

1. A method for a patient downgrade recommendation system of an intensive care unit (ICU), comprising:
for each patient of a plurality of selected patients of the ICU:
selecting (454) a set of downgrade criteria for the patient;
from a first, larger set of patient data of the patient accessible from a plurality of sources communicably coupled to the patient downgrade recommendation system, automatically compiling (458) a second, smaller set of patient data based on the set of downgrade criteria;
applying (456) a set of rules associated with the set of downgrade criteria to the second, smaller set of patient data to generate a downgrade recommendation on whether the patient may be downgraded from a first status to a second, lower status, the first status and second status based on one or more medical conditions of the patient;
generating (460) a graphical downgrade recommendation element summarizing the downgrade recommendation;
arranging (408) each graphical downgrade recommendation element of the plurality of selected patients in a single integrated view of a user interface (UI) of the patient downgrade recommendation system; and
displaying (408) the UI on a display device of the patient downgrade recommendation system.

2. The method of claim 1, wherein the first, larger set of patient data of each patient of the plurality of selected patients is distributed across one or more systems and/or databases communicatively coupled to a computer system of the ICU via a network, including one or more Electronic Health Record (EHR) systems.

3. The method of claim 2, further comprising:
in a first condition, in response to the second, smaller set of patient data of a patient meeting conditions imposed by the set of rules, generating a downgrade recommendation that the patient be downgraded; and
in a second condition, in response to the second, smaller set of patient data of the patient not meeting one or more of the conditions imposed by the set of rules, generating a downgrade recommendation that the patient not be eligible for a downgrade.

4. The method of claim 2, further comprising:
in response to clinical information of the patient data being unavailable, the clinical information relied on for applying a rule to the patient data:
displaying a textual indication in the UI that a downgrade recommendation of the patient is pending an availability of the clinical information that is unavailable;
periodically attempting to retrieve the clinical information;
in response to retrieving the clinical information, generating the downgrade recommendation of the patient; and
displaying the downgrade recommendation in the UI.

5. The method of claim 2, wherein arranging the graphical downgrade recommendation elements of the plurality of selected patients in the single integrated view of the UI further comprises displaying, for one or more patients of the plurality of selected patients, a downgrade alert indicating a contributing factor to a downgrade recommendation of the patient.

6. The method of claim 2, wherein arranging the graphical downgrade recommendation elements of the plurality of selected patients in the single integrated view of the UI further comprises displaying, for a patient of the plurality of selected patients, one or more of:
an alert generated for the patient by a patient management system coupled to the patient downgrade recommendation system via the network; and
a discharge milestone indicating a task pending completion for discharging the patient from the ICU.

7. The method of claim 2, further comprising structuring the set of rules associated with the set of downgrade criteria as a limited list of machine-implementable disqualification rules, each disqualification rule including a condition corresponding to no more than 3 downgrade criteria to be satisfied by patient data of the second, smaller set of patient data, and in response to one or more conditions not being satisfied, disqualifying the patient from being eligible for a downgrade,
wherein each disqualification rule is stored as a separate record in a database, and
wherein applying the set of rules includes:
retrieving disqualification rules corresponding to the set of downgrade criteria from the database;
applying the disqualification rules in parallel or in series to corresponding data of the second, smaller set of patient data; and
generating a downgrade recommendation based on whether the patient was disqualified by one or more of the disqualification rules.

8. The method of claim 7, wherein the second, smaller set of patient data is updated asynchronously, and the patient downgrade recommendation system periodically reapplies the disqualification rules to the second, smaller set of patient data to generate real-time updates to downgrade recommendations.

9. The method of claim 2, further comprising:
in response to a user selecting a selected graphical downgrade recommendation element of the UI of the patient downgrade recommendation system, generating a first display panel with information of a downgrade recommendation and/or controls associated with the selected graphical downgrade recommendation element;
in response to the user selecting a patient alert, discharge milestone, or downgrade alert of the UI, generating a second display panel with information and/or controls associated with the patient alert, discharge milestone, or downgrade alert; and
in response to the user of the patient downgrade recommendation system confirming a downgrade recommendation of a patient via a control of the first display panel:
initiating a transfer of the patient from the ICU to a receiving care unit;
displaying one or more discharge milestones and/or alerts for the patient in the UI;
displaying one or more downgrade readiness icons in the UI, indicating a readiness of the patient to be transferred;
periodically retrieving updated information of the one or more discharge milestones and/or alerts from one or more systems and/or databases communicatively coupled to the patient downgrade recommendation system via a network; and
automatically updating the one or more discharge milestones and/or alerts in the UI with the updated information.

10. The method of claim 2, wherein the graphical downgrade recommendation element includes one of:
an indication that the patient is ready for a transfer;
an indication that the patient is ready to downgrade to an intermediate/step-down unit;
an indication that the patient may possibly be ready to be downgraded;
an indication that the patient has met sub-specialty criteria and is ready to laterally transfer to another ICU;
an indication that the patient is not eligible for a downgrade.

11. The method of claim 2, wherein automatically compiling the second, smaller set of patient data based on the set of downgrade criteria further includes retrieving a plurality of clinical markers of the patient, each clinical marker of the plurality of clinical markers recorded at a different time, and including in the second, smaller set of patient data one of:
a most recent clinical marker of the plurality of clinical markers; and
a result of analyzing a trend in the plurality of clinical markers.

12. A patient downgrade recommendation system (220), comprising:
one or more processors (206); and
memory (204) storing instructions executable by the one or more processors to:
receive (458), at the patient downgrade recommendation system, a feed from an electronic health record database, where one or more values for each of a plurality of parameters for each of a plurality of patients are sent over the feed;
output (408), for display on a display device, a recommendation graphical user interface (GUI) that includes, for one or more patients of the plurality of patients, a downgrade recommendation recommending whether or not to downgrade the patient, where each downgrade recommendation is generated by applying a set of downgrade rules to a set of patient data obtained from the feed; and
display (410), on the recommendation GUI, for a selected patient of the one or more patients, a downgrade element indicating a contributing factor to a downgrade recommendation of the selected patient.

13. The system of claim 12, wherein updated values for each of the plurality of parameters are received in real-time via the feed and wherein each downgrade recommendation is updated at a predetermined frequency.

14. The system of claim 12, wherein the instructions are further executable to receive a notification that a transfer order has been received for a selected patient and, in response, update the downgrade recommendation on the recommendation GUI for the selected patient to indicate the transfer order.

15. The system of claim 12, wherein the instructions are further executable to:
in response to a first user input to the recommendation GUI, output, for display on the recommendation GUI, a first display panel including a list of all criteria considered in generating a selected downgrade recommendation; and
in response to a second user input to the recommendation GUI, output, for display on the recommendation GUI, a second display panel including additional information relating to a selected criterion considered in generating a selected downgrade recommendation.
